# EUROPÄISCHE PATENTSCHRIFT

(11) **EP 4 544 037 B1**
(45) Veröffentlichungstag und Bekanntmachung des Hinweises auf die Patenterteilung: **21.01.2026**
(21) Anmeldenummer: 23714578.4
(22) Anmeldetag: 29.03.2023
(51) Int. Cl.: C12N 15/10

(54) **CRISPR-CAS9 BASIERTE LINEARISIERUNG VON PLASMID DNA-TEMPLATEN**
CRISPR-CAS9-BASED LINEARIZATION OF PLASMID DNA TEMPLATES
LINÉARISATION BASÉE SUR CRISPR-CAS9 DE MODÈLES D'ADN PLASMIDIQUE

(43) Veröffentlichungstag der Anmeldung: 30.04.2025
(73) Patentinhaber: Wacker Chemie AG, 81671 München (DE)
(72) Erfinder: RICHTER, Hagen, 81737 München (DE); BORNHOEVD, Carsten, 81737 München (DE); CRONET, Philippe, 81737 München (DE); KUJAU, Marian, 81737 München (DE); WICH, Guenter, 81737 München (DE)
(74) Vertreter: Gross, Christine Dorothee
(86) Internationale Anmeldenummer: PCT/EP2023/058156
(87) Internationale Veröffentlichungsnummer: WO 2024/199650

(56) Entgegenhaltungen:
- EP-B1- 3 289 077
- EP-B1- 3 294 885
- CA-A1- 3 171 495
- US-A1- 2020 392 518
- US-B2- 10 106 800
- US-B2- 10 760 070

## Beschreibung

Die Erfindung betrifft ein Verfahren zur Herstellung eines linearen doppelsträngigen DNA-Moleküls, worin das lineare DNA-Molekül glatte Enden aufweist, und worin das lineare DNA-Molekül eine Poly T-Sequenz am 5' Ende des Matrizen-Strangs aufweist, umfassend die Schritte:
(1) Bereitstellen eines doppelsträngigen zirkulären DNA-Moleküls, umfassend auf dem Matrizen-Strang in der 3'- zu 5'-Richtung:
   (i) eine Templat-Sequenz, die operativ mit einem RNA-Polymerase-Promotor verbunden ist,
   (ii) eine Poly T-Sequenz,
   (iii) eine Nukleotidsequenz, dargestellt durch N₁N₂N₃, worin N₁ bis N₃ jeweils unabhängig voneinander G, T, A oder C oder ein Nukleotidanalogon davon darstellen, und
   (iv) ein Protospacer Adjacent Motif (PAM),
(2) Bereitstellen einer Führungs-RNA, umfassend eine Region, die komplementär ist zum 5'-Ende der Poly T-Sequenz (ii) und zur Nukleotidsequenz (iii), und eine Region, die mit einer Cas-Nuklease interagiert,
(3) Bereitstellen einer Cas-Nuklease vom Typ II,
(4) In Kontakt bringen des doppelsträngigen zirkulären DNA-Moleküls mit der Führungs-RNA und der CAS-Nuklease.

Ribonukleinsäuren (RNAs) spielen in vielen zellulären Prozessen eine entscheidende Rolle. Im zentralen Dogma der Biologie vermitteln sie die Übersetzung der DNA in Proteine und spielen mit messenger RNAs (mRNAs) und transfer RNAs (tRNAs) die Schlüsselrolle bei der Transkription und Translation. Aber auch viele andere Prozesse, vor allem auf regulatorischer Ebene, also der Feinabstimmung von biochemischen Reaktionen innerhalb und außerhalb von Zellen, werden durch RNAs maßgeblich gesteuert. Daher ist es nicht verwunderlich, dass RNAs ein Ziel von vielen Forschungsarbeiten sind, um diese Prozesse besser zu verstehen.

Neben grundlegenden Arbeiten zum allgemeinen Verständnis von RNAs hat sich vor allem in den letzten Jahren gezeigt, wie nützlich RNAs auch für therapeutische Anwendungen sind, wie z.B. die Impfstoffe gegen SARS-Cov2. Für solche Anwendungsgebiete ist es wichtig, dass geeignete Verfahren zur Verfügung stehen, um ausreichende Mengen an mRNA herstellen zu können. Diese Verfahren bedienen sich natürlich vorkommender Moleküle, beispielsweise RNA-Polymerasen, welche RNAs produzieren. Die synthetische Herstellung von RNAs durch DNA abhängige RNA-Polymerasen ist ein Verfahren, welches für Forschungs- und Industrieanwendungen routinemäßig eingesetzt wird. Bei diesem Verfahren werden eine DNA-Matrize, eine RNA-Polymerase, z.B. eine T7 RNA-Polymerase, Nukleotide wie ATP, CTP, GTP, UTP sowie geeignete Pufferbedingungen benötigt. Dann synthetisiert die Polymerase in Abhängigkeit vom DNA-Templat eine entsprechende komplementäre RNA. Die hergestellten RNAs können unterschiedlicher Art sein: mRNAs, *self-amplifying* RNAs (saRNAs), lange, nicht kodierende RNAs (lncRNAs), *guide* RNAs (gRNAs), *short interfering* RNAs (siRNA), *micro* RNAs (miRNAs), zirkuläre RNAs (circRNAs) und andere. Vor allem bei kürzeren RNAs kann industriell auch eine chemische Synthese verwendet werden. Für längere RNAs von über 100 Nukleotiden, beispielsweise mRNAs, ist eine chemische Synthese allerdings nicht ökonomisch, weswegen längere RNAs meist mittels einer enzymatischen *in* vitro-Transkription hergestellt werden.

In der großtechnischen Produktion wird für eine *in vitro-*Transkription in der Regel Plasmid-DNA als DNA-Templat verwendet, da Plasmid-DNA, anders als durch PCR hergestellte DNA-Template, mittels Fermentation in den notwendigen Mengen produziert werden kann. Die in sich geschlossene, zirkuläre Natur von Plasmiden führt bei einer RNA-Synthese zu einem theoretisch unendlichen langen RNA-Transkript, in der Praxis immer zumindest anteilig zu ungewollt heterogenen, verlängerten RNAs. Diese Verlängerungen wirken sich negativ auf die Effizienz des Transkriptionsprozesses aus, wodurch die Herstellungskosten steigen. In der Literatur werden zwei mögliche Ansätze beschrieben, um die ungewollten verlängerten RNA-Transkripte zu vermeiden.

Eine Lösung ist die klassische Nutzung von Restriktionsendonukleasen vom Typ II. Diese Enzyme erkennen eine spezifische DNA-Sequenz und führen in der Regel innerhalb oder nahe dieser Sequenz zu einem gezielten Schnitt im Doppelstrang der DNA. Mit Hilfe eines solchen Schnittes in der Plasmid-DNA wird eine so genannte *in vitro* Run-Off-Transkription ermöglicht, bei welcher die RNA-Polymerase am Doppelstrangbruch "abfällt" und erneut an einen Promoter binden und eine neue Syntheserunde starten kann. Für einen solchen Schnitt in der DNA-Matrize werden typischerweise klassische Restriktionsenzyme vom Typ II eingesetzt. Restriktionsenzyme der Untergruppe Typ IIP schneiden DNA an definierten Positionen innerhalb ihrer Erkennungssequenzen. Zu den Restriktionsenzymen vom Typ IIP gehören zum Beispiel EcoRI und *XbaI.* Alternativ können auch Enzyme der Untergruppe Typ IIS verwendet werden, die außerhalb ihrer Erkennungssequenzen schneiden. Mit beiden Restriktionsenzymklassen vom Typ II (Typ IIP und Typ IIS) lassen sich aus der zirkulären Plasmid-DNA lineare DNA-Fragmente erzeugen, die sich zur effizienteren RNA-Synthese einsetzen lassen. Zu beachten ist hierbei, dass das DNA-Templat keine weiteren Erkennungssequenzen für die jeweils verwendeten Restriktionsenzyme enthalten sollte. Somit ergibt sich eine Einschränkung bei der Templat-Sequenz bzw. ein aufwändigeres Design derselben. Das kann einzelne Basenpaarungen betreffen, aber auch ganze Codone des DNA-Templats oder Domänen der Zielproteine, beispielsweise Motive oder Strukturen. Zusätzlich prozessieren viele Typ II-Restriktionsenzyme, vor allem Enzyme des Typs IIS, die DNA mit einem Überhang, wobei so genannte klebrige Enden 5' oder 3' entstehen. Dabei ist besonders ein 3' Überhang ungünstig, da dieser durch die Promiskuität der RNA-Polymerase zu längeren Transkripten führen kann als ursprünglich geplant. Um diese ungewollten Transkripte zu verhindern, müsste der Überhang im Anschluss an den Restriktionsschritt abgebaut werden. Damit ergeben sich in der Planung und der Erzeugung der DNA-Template deutlich mehr Schritte und es steigt der entsprechende Entwicklungsaufwand.

Die zweite Lösung zur Vermeidung von zu langen RNA-Transkripten ist die Verwendung von Rho-unabhängigen Terminationssignalen. Im Gegensatz zu einer Verwendung von Restriktionsendonukleasen wird das DNA-Templat hierbei nicht linearisiert. Rho-unabhängige Terminatoren sind üblicherweise sekundäre Strukturen innerhalb der synthetisierten Ziel-RNA und verhindern das Entstehen von zu langen RNA-Transkripten mittels Syntheseabbruch. Nach einem erfolgten Syntheseabbruch kann die Polymerase, ähnlich wie bei der Run-Off-Transkription, erneut einen Promoter binden und eine neue Syntheserunde starten. Dabei bleibt eine hohe Transkriptionsrate erhalten, es werden allerdings auch Sequenzbereiche angefügt, welche unerwünscht sind, **z.B.** die Terminator-Sequenz. Um diese unerwünschten Elemente zu entfernen, können neben RNA-schneidenden Enzymen, **z.B.** RNasen, auch katalytisch aktive Nukleinsäuren eingesetzt werden. Zu diesen zählen zum Beispiel DNAzyme, aber auch sogenannte Ribozyme. Ribozyme sind strukturierte RNA-Moleküle, welche *in trans* (von außen kommend) oder *in cis* (von der Sequenz kommend) RNAs an definierten Stellen schneiden. Hierfür ist in der Regel eine bestimmte RNA-Struktur und ein zweiwertiges Metallion nötig, typischerweise Mg²⁺. Dadurch erfolgt ein nucleophiler Angriff an der Zielsequenz, wodurch diese gespalten wird und im Falle einer RNA-Synthese überschüssige Elemente entfernt werden können. Da die T7 RNA-Polymerase ebenfalls auf Mg²⁺ Ionen angewiesen ist, wird die Ribozym-Aktivität innerhalb der Synthese-Reaktion einen Einfluss auf die Effizienz der *in* vitro-Transkription haben, da eine Konkurrenz um die Mg²⁺ Ionen besteht. Damit ist eine weitere Optimierung der Reaktionsbedingungen erforderlich, was mit Effizienzeinbußen einhergehen kann. Beim Entwurf entsprechender Sequenzen mit einem Cis-Ribozym ist darauf zu achten, dass ein Ribozym gefolgt von mindestens einer Terminator-Sequenz in die Ziel-RNA eingebaut wird. Entsprechend wird der gesamte Prozess zur Herstellung von DNA-Templaten für die mRNA-Herstellung teurer und somit weniger wirtschaftlich.

Für die enzymatische Herstellung von funktionalen reifen mRNAs und saRNAs, welche in Säugetierzellen aktiv sein sollen, ist es zwingend notwendig, dass die RNA einen Poly A-Anhang aufweist. Dieser Poly A-Anhang erhöht die Stabilität der RNA und spielt auch während der Translation in der eukaryotischen Zielzelle eine entscheidende Rolle. Der Poly A-Anhang wird in der Zielzelle durch Poly A-bindende Proteine erkannt und gebunden. Durch die gebundenen Proteine kommt es zur Interaktion des 3' Endes der mRNA mit den Proteinen der Translationsmaschinerie auf dem 5' Ende der mRNA. Dieses komplexe Zusammenspiel erhöht neben der Stabilität der RNA auch die Translationseffizienz. Daraus ergibt sich eine Schlüsselrolle des Poly A-Anhangs innerhalb der reifen mRNA. Um eine möglichst natürliche mRNA bzw. saRNA darzustellen, sollte der Poly A-Anhang möglichst lang (> 100 A-Nukleotide) und idealerweise homogen sein (ohne andere Basen; C, G oder U). Man spricht dann von einem unverdeckten Poly A-Anhang am 3' Ende der RNA.

Zwei Arten zur Produktion von RNA-Molekülen mit einheitlichem Poly A-Anhang sind derzeit üblich. Zum einen kann nach der Synthese der Ziel-RNA-Sequenz der Poly A-Anhang über eine Poly A-Polymerase generiert werden. Im Falle dieses enzymatischen Schrittes wird die hergestellte RNA zunächst gereinigt, bevor durch Zugabe einer Poly A-Polymerase und ATP als Substrat ein Poly A-Anhang synthetisiert wird. Die Dauer der Reaktion bestimmt die durchschnittliche Länge der Poly A-Anhänge. Diese enzymatische Synthese funktioniert in der Regel sehr gut, birgt aber auch einige Nachteile. So ist es mit dieser Technik nicht möglich, eine klar definierte Länge des Poly A-Anhangs zu erzeugen, und es entsteht ein Produkt mit inhomogenen RNA-Molekülen von normal-verteilter Länge. Für eine industrielle Nutzung der so hergestellten RNA-Moleküle sind daher weitere Prozessschritte zur Anreicherung bzw. zur Reinigung der RNAs nötig, wodurch die Ausbeuten sinken, und die Herstellungskosten steigen.

Demgegenüber steht eine Methode, bei der die Kodierung des Poly A-Anhangs direkt durch das DNA-Templat erfolgt, welches als Vorlage zur Herstellung der RNA dient. Für die Polyadenylierung wird hierfür beim Design der Templat-DNA darauf geachtet, dass die doppelsträngige DNA auf dem Matrizen-Strang das nötige Poly T-Motiv enthält. Der Vorteil dieses Verfahrens liegt darin, dass innerhalb eines Reaktionsschrittes ein wesentlich definierteres Produkt mit einem Poly A-Anhang mit einheitlicher Länge entsteht.

Wenn PCR-Produkte als Templat für die Synthese von RNA-Molekülen mit homogenen und unverdeckten Poly A-Anhängen genutzt werden, kann der Poly A-Anhang direkt in den für die PCR verwendeten Primern kodiert werden. Für die Herstellung eines DNA-Templats mittels PCR kann ein reverser Primer verwendet werden, der neben der komplementären Sequenz am 3' Ende eine Abfolge von bis zu 120 Adeninen oder auch mehr enthält. In der PCR wird dieser Anhang an die kodierende DNA angefügt und steht somit für die *in vitro-*Transkription zur Verfügung. Da sich PCR-Reaktionen technisch meist nur in kleinen Volumina bis zu wenigen hundert Mikrolitern durchführen lassen, würden für eine großtechnische Verarbeitung zur RNA mehrere hundert PCR-Reaktionen benötigt. Eine Hochskalierung des PCR-Ansatzes durch ein sogenanntes Scale-Out, bei dem mehrere Reaktionen parallel durchgeführt werden, ist aufgrund des hohen Arbeits- und Kostenaufwands im industriellen Maßstab aktuell nicht wirtschaftlich.

Wenn Plasmid-DNA als Templat für die *in* vitro-Transkription von RNA verwendet wird, so ist eine fermentative Produktion der Plasmide, eine Aufreinigung der pDNA und anschließend eine Linearisierung der pDNA notwendig. Nach der Linearisierung muss eine erneute Aufreinigung der linearisierten pDNA erfolgen, um eine möglichst reine DNA-Matrize für die *in* vitro-Transkription bereitzustellen, wodurch die Komplexität und die Kosten des Verfahrens steigen. Wie bereits zuvor angemerkt liegt ein Nachteil bei der Verwendung von Typ IIP Restriktionsenzymen darin, dass diese Enzyme keinen freien unverdeckten Poly A-Anhang ergeben, da die Erkennungssequenz von Typ IIP Restriktionsenzymen nahezu immer auch andere Nukleotide wie C, G, oder T enthält. Zwar könnte diesbezüglich die Nutzung von Typ IIS Enzymen Abhilfe schaffen, da diese außerhalb ihrer Erkennungssequenz schneiden und so der gewünschte unverdeckte Poly A-Anhang geschaffen werden könnte. Allerdings führt die Verwendung von Typ IIS Enzymen üblicherweise zu 3' bzw. 5' Überhängen, die wie oben beschrieben, zunächst abgebaut werden müssten.

Neben der Verwendung von Restriktionsenzymen ist es auch möglich, Ribozyme zur Linearisierung der Templat-DNA zu verwenden. Auch diese können so entworfen werden, dass ein homogener und freier Poly A-Anhang entstehen kann. Ribozyme werden bevorzugt so entworfen, dass sie direkt am Ende der Poly A-Sequenz schneiden. Der Nachteil der Ribozyme liegt darin, dass bei *trans* Ribozymen eine weitere RNA bzw. bei *cis* Ribozymen eine deutlich längere RNA hergestellt werden muss. Dieses Vorgehen erhöht den Produktionsaufwand und entsprechend die Kosten.

CRISPR *(Clustered Regularly Interspaced Short Palindromic Repeats)* und CRISPR-assoziierte (Cas) Nukleasen wurden als Teile bakterieller Immunsysteme identifiziert. Da Cas-Nukleasen DNA-Elemente präzise an einer bestimmten Stelle schneiden können, wurden CRISPR-Cas-Systeme als effiziente Geneditierungswerkzeuge weiterentwickelt, hauptsächlich für Anwendungen im Bereich der Genomeditierung, und haben in diesem Bereich zu einer Revolution der Molekular- und Mikrobiologie geführt (Hille et al., Cell 172, Issue 6, 1239-1259, 2018; Plagens et al. FEMS Microbiology Reviews, 39(3):442-63, 2015; Richter et al. Int J Mol Sci. 2013 Jul; 14(7): 14518-14531).

Wird eine bakterielle Zelle von einer fremden Nukleinsäure infiziert, beispielsweise Phagen-DNA, Transposons, pDNA oder RNA, reagiert das Bakterium mit einer Immunantwort, um die fremde DNA oder RNA zu eliminieren. Das Besondere an der CRISPR-Cas-Immunität ist, dass es sich um eine adaptive Immunität handelt, was bedeutet, dass die Bakterienzelle diese Art von Immunantwort erlernt. Während einer Infektion wird ein kurzes DNA-Fragment der fremden Nukleinsäure für das Immunsystem verwendet, wodurch später bei einer gleichen Art der Infektion eine schnellere Antwort induziert werden kann. Dieses DNA-Fragment wird innerhalb des genetischen Lokus für das CRISPR-Cas System im CRISPR-array eingebaut und wird dort als *spacer* bezeichnet. Diese spacer-Sequenz wird später Teil der Führungs-RNA (gRNA) bzw. CRISPR-RNA (crRNA) und trägt somit zur Sequenzspezifität bei. Im Rahmen dieser bakteriellen Immunabwehr wird die Cas-Nuklease durch die crRNA zu einer spezifischen Zielsequenz auf der fremden Nukleinsäure dirigiert. Für die Erkennung der Sequenz ist zum einen die Komplementarität der crRNA mit der fremden Ziel-DNA nötig, und zum anderen eine kurze Signalsequenz in direkter Nähe der Ziel-DNA, die als *Protospacer Adjacent Motif* (PAM) bezeichnet wird. Die PAM-Sequenz besteht je nach CRISPR-Cas System aus einer unterschiedlichen Anzahl an spezifischen Nukleotiden und ist die initiale Erkennungssequenz für die Cas-Nuklease auf der zu schneidenden DNA. Beispielsweise erkennt die Cas-Nuklease Cas9 von *Streptococcus pyogenes* (SpCas9) die PAM-Sequenz 5'-NGG-3'. Ohne die PAM-Sequenz in direkter Nähe zur Zielsequenz erfolgt kein Schnitt der fremden DNA. Die fremde Zielsequenz wird nach der Erkennung durch den Ribonukleoprotein-Komplex aus Cas Nuklease und crRNA spezifisch geschnitten und im Folgenden kann die fremde DNA durch weitere zelluläre Nukleasen abgebaut werden. Nur die passende Kombination aus PAM-Sequenz und Zielsequenz in entsprechender räumlicher Nähe führen zur Schneideaktivität der Cas-Nuklease. Da eine spezifische Kombination aus PAM und Zielsequenz in der Regel im Genom der Bakterien nicht vorhanden ist, wird eine Autoimmunreaktion, d.h. das Schneiden der eigenen DNA verhindert.

Es gibt unterschiedliche CRISPR-Cas-Systeme, wobei sich diese insbesondere in der Art und Anzahl der Proteine unterscheiden, die erforderlich ist, damit der CRISPR-Komplex an der richtigen Stelle der Zielsequenz binden und schneiden kann. Besonders gut verstanden sind die Systeme von Typ II-A, welche für die Interferenz mit der Ziel-DNA das Cas9-Protein als EffektorNuklease verwendet. Neben dem Namensgebenden CRISPR Array, welcher die verschiedenen crRNAs kodiert, umfassen die Typ II-Systeme eine trans-aktivierende RNA (tracrRNA) die Adaptionsproteine Cas1, Cas2 und Cas4, sowie die Effektornuklease Cas9. Die tracrRNA bildet ein Hybrid mit der vom CRISPR Array kodierten crRNA. Dieses Hybrid wiederum wird spezifisch von Cas9 gebunden. Der crRNA Anteil des Hybrid-Konstruktes leitet Cas9 zur Ziel-DNA. Das zweiteilige System aus crRNA und tracrRNA ist für eine Anwendung in der Molekularbiologie relativ umständlich, daher werden die crRNA und die tacrRNA oft mit einem Linker verbunden. Diese resultierende RNA nennt man sgRNA (single guide RNA).

Eine Aufgabe der Erfindung ist es, ein vereinfachtes bzw. verbessertes Verfahren zur Linearisierung zirkulärer DNA-Moleküle zur Verfügung zu stellen, welche als DNA-Template für die enzymatische RNA-Synthese verwendet werden können. Dazu soll das linearisierte DNA-Molekül über glatte Enden verfügen und eine Poly T-Sequenz am 5' Ende des Matrizen-Strangs aufweisen. Insbesondere soll das Verfahren linearisierte DNA-Moleküle bereitstellen, welche die Synthese von RNAs mit einem homogenen und unverdeckten Poly A-Anhang ermöglichen. Eine andere Aufgabe der Erfindung besteht darin, ein Verfahren bereitzustellen, welches weniger Verfahrensschritte benötigt als die im Stand der Technik offenbarten Verfahren. Eine andere Aufgabe besteht darin, ein Verfahren bereitzustellen, welches lineare DNA-Moleküle ergibt, die eine verbesserte Homogenität hinsichtlich der Länge des Poly-A-Anhangs aufweisen. Eine andere Aufgabe ist die Bereitstellung eines Verfahrens, welches effizienter bzw. kostengünstiger durchgeführt werden kann als die im Stand der Technik beschrieben Verfahren.

Die Aufgabe wird gelöst durch ein Verfahren zur Herstellung eines linearen doppelsträngigen DNA-Moleküls, worin das lineare DNA-Molekül glatte Enden aufweist, und worin das lineare DNA-Molekül eine Poly T-Sequenz am 5' Ende des Matrizen-Strangs aufweist, umfassend die Schritte:
(1) Bereitstellen eines doppelsträngigen zirkulären DNA-Moleküls, umfassend auf dem Matrizen-Strang in der 3'- zu 5'-Richtung:
   (i) eine Templat-Sequenz, die operativ mit einem RNA-Polymerase-Promotor verbunden ist,
   (ii)eine Poly T-Sequenz,
   (iii)eine Nukleotidsequenz, dargestellt durch N₁N₂N₃, worin N₁ bis N₃ jeweils unabhängig voneinander G, T, A oder C oder ein Nukleotidanalogon davon darstellen, und
   (iv) ein Protospacer Adjacent Motif (PAM),
(2) Bereitstellen einer Führungs-RNA, umfassend eine Region, die komplementär ist zum 5'-Ende der Poly T-Sequenz (ii) und zur Nukleotidsequenz (iii), und eine Region, die mit einer Cas-Nuklease interagiert,
(3) Bereitstellen einer Cas-Nuklease vom Typ II,
(4) In Kontakt bringen des doppelsträngigen zirkulären DNA-Moleküls mit der Führungs-RNA und der CAS-Nuklease.

Eine Anwendung von CRISPR-Cas Systemen im Bereich der Linearisierung von Plasmid-DNA, insbesondere als Templat für eine *in* vitro-Transkription von RNA, ist bisher noch nicht bekannt. Durch eine Kombination einer Führungs-RNA, einer Cas-Nuklease, beispielsweise Cas9, und einem doppelsträngigen zirkulären DNA-Molekül kann ein CRISPR-Cas System für die effiziente und homogene Linearisierung von Plasmid-DNA eingesetzt werden.

Durch die flexible Gestaltungsmöglichkeit der Führungs-RNA kann das Verfahren zur Linearisierung flexibel für sämtliche doppelsträngige zirkuläre DNA-Moleküle verwendet werden, wobei ein spezifischer Schnitt an einer vorab ausgewählten Stelle der DNA möglich ist.

Durch die flexible Gestaltungsmöglichkeit des PAM kann das Verfahren zur Linearisierung flexibel mit unterschiedlichen Cas-Nukleasen durchgeführt werden.

Cas-Nukleasen des Typs II, beispielsweise Cas9, bilden beim Schnitt des Doppelstrangs im Zielmolekül sogenannte "glatte" oder "stumpfe" DNA-Enden, **d.h.** DNA-Enden ohne **3'-** oder **5'-**Überhang. Derartige DNA-Matrizen mit glatten Enden sind für die RNA-Synthese besonders gut geeignet. Bei der mRNA-Synthese ist es wünschenswert, dass die resultierende mRNA einen homogenen und unverdeckten Poly A-Anhang mit möglichst definierter Länge besitzt. Je homogener die Poly A-Anhänge der reifen mRNA sind, **d.h.** desto weniger andere Basen als Adenin enthalten sind, desto einfacher und effizienter ist die Bindung des Poly A-bindenden Proteins. Die Bindung dieses Proteins wiederum ist entscheidend für eine effektive Translation der reifen mRNA innerhalb der Zielzelle. Negative Effekte von mRNAs, die nicht direkt mit Adenin-Resten bzw. mit unterschiedlichen Basen enden, wie sie beispielsweise bei der Verwendung von vielen Typ II-Restriktionsenzymen entstehen, können so vermieden werden. Damit ermöglicht das erfindungsgemäße Verfahren die Erzeugung von linearen DNA-Templaten, die zur Herstellung einer natürlichen, reifen RNA, beispielsweise einer mRNA, besonders gut geeignet sind, die bei therapeutischen Anwendungen Vorteile aufweist, **z.B.** eine verbesserte Stabilität, längere Halbwertzeit oder eine höhere Translationseffizienz.

Das erfindungsgemäße Verfahren bietet einen hohen Freiheitsgrad beim Sequenzdesign und eine absolute Sequenzunabhängigkeit, **d.h.** es gibt keine Einschränkungen bei Nukleotiden sowie Sequenz- und Strukturmotiven.

Das erfindungsgemäße, auf CRISPR-Cas basierende Verfahren ist unabhängig von Methylierungen und anderen Modifikationen der DNA, wie es bei vielen Restriktionsenzymen der Fall ist.

Erfindungsgemäß kann eine Linearisierung des doppelsträngigen zirkulären DNA-Moleküls, **d.h.** des DNA-Templats, schon während der Vervielfältigung der Plasmid-DNA, also beispielsweise innerhalb der bakteriellen Produktionszelle bzw. vor dem Zellaufschluss erfolgen. Dies bietet den Vorteil, dass die Anzahl der Prozessschritte reduziert werden kann. So kann am Ende der pDNA-Fermentation bereits in der Zelle die Linearisierung durch eine Cas-Nuklease, beispielsweise Cas9, erfolgen. Die lineare DNA kann nach Abschluss der Fermentation und intrazellulären Linearisierung mittels herkömmlicher Methoden aufgereinigt werden, eine separate Aufarbeitung des doppelsträngigen zirkulären DNA-Moleküls entfällt. Anschließend kann das lineare DNA-Templat direkt in einer *in vitro-*Transkription zur Produktion von RNA, beispielsweise mRNA mit einem unverdeckten Poly A-Anhang verwendet werden.

Das erfindungsgemäß hergestellte lineare doppelsträngige DNA-Molekül weist glatte Enden und eine Poly T-Sequenz am 5' Ende des Matrizen-Strangs auf. Solche linearen DNA-Moleküle sind besonders gut geeignet als DNA-Template für eine *in* vitro-Transkription. Bei der *in* vitro-Transkription handelt es sich um eine enzymatische Katalyse, bei welcher *in vitro* eine RNA hergestellt wird, dabei wird mit einer DNA-abhängigen RNA-Polymerase in Abhängigkeit einer DNA-Matrize die dazu komplementäre RNA synthetisiert. Zur Synthese werden zudem Ribonukleotide (NTPs), ein Puffersystem, z.B. TRIS-HCl, Magnesium-Ionen, z.B. MgCl₂ und optional ein RNase-Inhibitor, eine anorganische Pyrophosphatase und Spermidin benötigt. Typischerweise werden monomere RNA-Polymerasen verwendet, welche in der Regel Bakteriophagen-Enzyme sind. Die am häufigsten verwendeten Polymerasen sind zum Beispiel die T7- RNA-Polymerase (Uniprot: P00573) oder die T3-RNA-Polymerase (Uniprot: Q778M8).

### Definitionen

Sofern es nicht anderweitig definiert ist, haben die hierin verwendeten technischen und wissenschaftlichen Fachbegriffe die gleiche Bedeutung, wie sie von einem Fachmann auf dem Gebiet der vorliegenden Erfindung üblicherweise verstanden wird. Jedes technische Merkmal, das bei den folgenden Definitionen erwähnt wird, kann auf jede Ausführungsform der Erfindung angewendet werden.

Die Begriffe Nukleinsäuresequenz, Nukleotidsequenz, DNA-Sequenz und RNA-Sequenz sind einem Fachmann bekannt und beziehen sich auf eine individuelle und bestimmte Abfolge von Nukleotiden. Die Begriffe DNS und DNA sowie die Begriffe RNS und RNA werden im Rahmen dieser Erfindung synonym verwendet.

Die Begriffe Polynukleotid und Nukleinsäure werden hierin austauschbar verwendet und beziehen sich auf eine Abfolge von Nukleotiden beliebiger Länge, entweder Ribonukleotide oder Desoxyribonukleotide. Diese Begriffe umfassen einzelsträngige und doppelsträngige DNA oder RNA, genomische DNA, cDNA, mRNA, saRNA, gRNA, siRNA, miRNA oder circRNA, welche Purin- und Pyrimidin-Basen, Nukleotidanaloga oder auch andere natürlich, chemische oder biochemisch modifizierte, nicht-natürliche oder derivatisierte Nukleotid-Basen umfassen kann.

Ein Nukleotidanalogon bezieht sich auf eine chemische Verbindung, die strukturell und funktionell dem Nukleotid ähnlich ist, d.h. das Nukleotidanalogon kann von einer Polymerase als Substrat erkannt werden. Unter Nukleotidanaloga versteht man hierin insbesondere nativ und nicht-nativ vorkommende Varianten der natürlich vorkommenden Nukleotide Adenosin, Cytosin, Thymidin, Guanosin und Uridin, beispielsweise chemisch derivatisierte Nukleotide mit nicht-nativ vorkommenden funktionellen Gruppen, die an das natürlich vorkommende Nukleotid angefügt oder aus ihm entfernt werden, oder die die natürlich vorkommenden funktionellen Gruppen eines Nukleotids ersetzen. Beispiele für Nukleotidanaloga umfassen N1-Methylpseudouridin, 5-Methoxyuridin, 5-Methylcytidin, Pseudouridin, N4-Acetylcytidin und N6-Methyladenin.

Unter einer Poly T-Sequenz wie hierin verwendet ist eine Abfolge von Thymin-Nukleotiden zu verstehen, worin bevorzugt keine anderen Nukleotide enthalten sind, d.h. kein Adenin, Guanin oder Cytosin.

Unter einer Poly A-Sequenz wie hierin verwendet ist eine Abfolge von Adenin-Nukleotiden zu verstehen, worin bevorzugt keine anderen Nukleotide enthalten sind, d.h. kein Thymin, Guanin oder Cytosin.

Eine Poly T-Sequenz bzw. eine Poly A-Sequenz kann in zwei oder mehr unterschiedliche Segmente mit jeweils mindestens etwa 40 Thymin- bzw. Adenin-Nukleotiden segmentiert sein. Die einzelnen Segmente können jeweils von Sequenzen von etwa 10 - 20 Nukleotiden Länge unterbrochen sein, die keine Poly T bzw. Poly A-Sequenzen sind, und auch G, C und U enthalten können. Eine solche Segmentierung kann eine verbesserte Stabilität der Poly T-Sequenz oder Poly A-Sequenz bewirken.

Unter einem unverdecktem Poly A-Anhang einer RNA ist hierin eine endständige Poly A-Sequenz zu verstehen, bei welcher auf die Abfolge von Adenin-Nukleotiden am 3' Ende der RNA keine anderen Nukleotide außer Adenin folgen, also kein Cytosin, Uracil oder Guanin.

Ein Poly A-Anhang einer RNA aus einer *in* vitro-Transkription wird hierin als homogen bezeichnet, wenn dieser neben dem unverdecktem 3' Ende eine enge Größenverteilung um eine definierte Ziellänge aufweist. Bevorzugt weist die durchschnittliche Abweichung zur Ziellänge nicht mehr als 10% bezogen auf die Anzahl der Nukleotide auf, mehr bevorzugt nicht mehr als 5% und besonders bevorzugt weniger als 5%. Ein segmentierter Poly A-Anhang gilt als homogen, wenn die Gesamtheit aller Segmente zusammen die oben genannte durchschnittliche Abweichung zur Ziellänge der Gesamtheit der Segmente aufweist. Eine Segmentierung kann eine Stabilisierung des gesamten Poly A-Anhangs bewirken. Auch bei einem segmentierten Poly A-Anhang einer RNA ist es von Vorteil, dass das 3' gelegene Segment des Poly A-Anhanges unverdeckt ist, um eine hohe Translationseffizienz zu gewährleisten.

Unter einem Matrizen-Strang einer doppelsträngigen DNA versteht man den DNA-Strang, welcher während einer Transkription von einer RNA-Polymerase abgelesen wird, um die RNA zu synthetisieren.

Unter einer Templat-Sequenz oder Templat-DNA bzw. einem DNA-Templat versteht man die DNA-Sequenz, die für eine *in vitro-*Transkription verwendet wird. Dabei dient diese Sequenz als Vorlage für eine DNA-abhängige RNA-Polymerase, um entsprechend dieser Sequenz eine RNA zu synthetisieren.

Eine Templat-Sequenz kann eine Nukleinsäure umfassen, welche für ein Antigen, beispielsweise eine Tumorantigen, ein virales oder bakterielles Antigen, ein therapeutisches Protein oder für andere Proteine wie z.B. Wachstumsfaktoren oder Transkriptionsfaktoren kodiert.

Wie hierin verwendet, ist eine Promotor-Sequenz oder ein Promotor ein DNA-regulatorischer Bereich, der in der Lage ist, eine RNA-Polymerase zu binden und die Transkription einer stromabwärts (in 3'-Richtung) gelegenen, kodierenden oder nicht-kodierenden Sequenz auszulösen. Geeignete Promotoren können von beliebigen Organismen abgeleitet werden, einschließlich prokaryotischen und eukaryotischen Organismen.

Wie hierin verwendet, bedeutet operativ mit einem Promotor verbunden, dass der Promotor die Transkription einer DNA bewirkt oder reguliert, die für ein Gen kodiert, wie beispielsweise das Gen, das durch die Templat-Sequenz kodiert wird, oder das Gen, das für die Führungs-RNA kodiert, oder das Gen, das für eine Cas-Nuklease vom Typ II kodiert. Der Promotor kann ein nativer Promotor sein, d.h. ein Promoter, der in der Zelle, in die der Vektor eingeführt wird, vorliegt. In einer Ausführungsform ist der Promotor ein induzierbarer Promotor, d.h. der Promotor wird reguliert, um eine induzierbare Transkription eines Gens zu ermöglichen, wie beispielsweise des Gens, das für die Führungs-RNA kodiert, oder des Gens, das für eine Cas-Nuklease vom Typ II kodiert.

Der Promotor kann ein konstitutiv aktiver Promotor sein, d.h. ein Promotor, der sich konstitutiv in einem aktiven Zustand befindet, oder es kann ein induzierbarer Promotor sein, d.h. ein Promotor, dessen Zustand durch einen externen Stimulus kontrolliert wird, bzw. vom inaktiven in einen aktiven Zustand gebracht wird. Durch einen Stimulus bzw. Induktor wird die Expression eines Zielgens induziert, also freigeschaltet. Diese Methode wird bevorzugt verwendet, wenn die Expression bzw. Überexpression eines Zielgens negative Auswirkungen auf den Produktionsorganismus oder auf ein Verfahren hat. Ein solcher externen Stimulus kann beispielsweise eine bestimmte Temperatur, eine Verbindung oder ein Protein sein.

Beispiele für induzierbare Promotoren umfassen den Lac-Promotor, der durch IPTG induzierbar ist (Jacob und Monod, Volume 3, Issue 3, Journal of Molecular Biology, June 1961, Pages 318-356, 1961), den Tetracyclin-regulierten Promotor (Gossen et al. 1995 Science 268 (5218): 1766-9.), den Rhamnose-induzierbaren Promotor (Kelly et al., ACS Synth. Biol.; 2016, 5 Pages 1136-1145),und den Arabinose-Promotor, der durch Arabinose induzierbar ist (Guzman et al., J Bacteriol. 1995, Jul;177(14):4121-30.).

Der Begriff *Protospacer Adjacent Motif* oder PAM beschreibt eine Signalsequenz aus etwa 2 bis 8 Basenpaaren, die die spezifische Erkennungnsstelle für eine Cas-Nuklease oder für einen Multiprotein Cas-Effektor Komplex (z.B. CASCADE oder CMR) auf der zu schneidenden DNA darstellt. Ohne Vorliegen einer PAM-Signalsequenz erfolgt kein Schnitt der Ziel-DNA. Die Länge und die Basensequenz der PAM-Sequenz unterscheiden sich je nach CRISPR-Cas System und Cas-Nuklease sowie dem dazugehörigen Organismus, in dem die Cas-Nuklease natürlicherweise vorkommt. Beispielsweise ist die PAM-Sequenz, die mit der Cas9-Nuklease von *Streptococcus pyogenes* assoziiert ist (SpCas9), 5'-NGG-3', wobei "N" ein beliebiges Nukleotid ist, also A, T, G oder C sein kann, gefolgt von zwei Guanin-Nukleotiden. Weitere Spezies und die damit assoziierten PAM-Sequenzen sind dem Fachmann bekannt und im Stand der Technik beschrieben (Fonfara et al. Nucleic Acids Research, 2014, Vol. 42, No. 4 2577-2590).

Eine Führungs-RNA, gRNA oder *guide* RNA ist eine spezifische RNA-Sequenz, an die eine Cas-Nuklease binden kann und die diese Cas-Nuklease zur Templat-DNA leitet. Die Führungs-RNA hybridisiert spezifisch an eine bestimmte Zielstelle der Templat-DNA, wo die Cas-Nuklease die Templat-DNA schneidet. Die Führungs-RNA kann zweiteilig sein und ein Hybrid aus crRNA und tracrRNA darstellen. Die Führungs-RNA kann alternativ eine einzelne sgRNA sein *(single guide* RNA), worin die wesentlichen Bereiche der crRNA und tracrRNA miteinander verbunden sind, so dass eine durchgängige chimäre RNA entsteht. Eine sgRNA imitiert die Haarnadelstruktur des crRNA::tracrRNA-Duplex und ist ebenfalls in der Lage, eine Cas-Nuklease zu binden und zur Zielsequenz auf der Templat-DNA zu dirigieren. Die Führungs-RNA wird gestaltet anhand der Sequenzinformation der Templat-DNA, die geschnitten werden soll.

Komplementär oder Komplementarität, wie hierin verwendet, beschreibt die Beziehung zwischen zwei DNA- oder RNA-Nukleinsäureregionen, welche auf Grund ihrer Nukleobasen nach Watson-Crick zueinander passen und Wasserstoffbrücken untereinander eingehen, d.h. hybridisieren. Im Stand der Technik sind beispielsweise Standard-Watson-Crick-Basenpaarungen bekannt: Adenin (A) paart sich mit Thymidin (T), Adenin (A) paart sich mit Uracil (U), und Guanin (G) paart sich mit Cytosin (C).

Unter homologen Genen bzw. homologen Sequenzen ist hierin zu verstehen, dass die DNA-Sequenzen dieser Gene bzw. DNA-Abschnitte zu mindestens 70%, bevorzugt zu mindestens 80%, bevorzugt zu mindestens 90% und besonders bevorzugt zu mindestens 95% identisch sind. Der Grad der DNA-Identität wird durch das Programm "nucleotide blast", zu finden auf der Seite http://blast.ncbi.nlm.nih.gov/, bestimmt, welches auf dem blastn-Algorithmus basiert. Als Algorithmus-Parameter für ein Alignment zweier oder mehrerer Nukleotidsequenzen wurden die voreingestellten Parameter genutzt. Die voreingestellten generellen Parameter sind: Max target sequences = 100; Short queries = "Automatically adjust parameters for short input sequences"; Expect Threshold = 10; Word size = 28; Automatically adjust parameters for short input sequences = 0. Die entsprechenden voreingestellten Scoring Parameter sind: Match/Mismatch Scores = 1,-2; Gap Costs = Linear.

Unter einer homologen Aminosäuresequenz ist eine Sequenz zu verstehen, die zu mindestens 70%, bevorzugt mindestens 80%, bevorzugt mindestens 90% und besonders bevorzugt mindestens 95% identisch ist, wobei jede Änderung in der homologen Sequenz ausgewählt ist aus Insertion, Addition, Deletion und Substitution einer oder mehrerer Aminosäuren. Für den Vergleich von Proteinsequenzen wird das Programm "protein blast", auf der Seite http://blast.ncbi.nlm.nih.gov/, genutzt. Dieses Programm greift auf den blastp-Algorithmus zurück. Als Algorithmus-Parameter für ein Alignment zweier oder mehrerer Proteinsequenzen wurden die voreingestellten Parameter genutzt. Die voreingestellten generellen Parameter sind: Max target sequences = 100; Short queries = "Automatically adjust parameters for short input sequences"; Expect Threshold = 10; Word size = 3; Automatically adjust parameters for short input sequences = 0. Die voreingestellten Scoring Parameter sind: Matrix = BLOSUM62; Gap Costs = Existence: 11 Extension: 1; Compositional adjustments = Conditional compositional score matrix adjustment.

### Ausführliche Beschreibung der Erfindung

In einem Aspekt betrifft die vorliegende Erfindung ein Verfahren zur Herstellung eines linearen doppelsträngigen DNA-Moleküls, worin das lineare DNA-Molekül glatte Enden aufweist, und worin das lineare DNA-Molekül eine Poly T-Sequenz am 5' Ende des Matrizen-Strangs aufweist, umfassend die Schritte:
(1) Bereitstellen eines doppelsträngigen zirkulären DNA-Moleküls, umfassend auf dem Matrizen-Strang in der 3'- zu 5'-Richtung:
   (i) eine Templat-Sequenz, die operativ mit einem RNA-Polymerase-Promotor verbunden ist,
   (ii) eine Poly T-Sequenz,
   (iii)eine Nukleotidsequenz, dargestellt durch N₁N₂N₃, worin N₁ bis N₃ jeweils unabhängig voneinander G, T, A oder C oder ein Nukleotidanalogon davon darstellen, und
   (iv) ein Protospacer Adjacent Motif (PAM),
(2) Bereitstellen einer Führungs-RNA, umfassend eine Region, die komplementär ist zum 5'-Ende der Poly T-Sequenz (ii) und zur Nukleotidsequenz (iii), und eine Region, die mit einer Cas-Nuklease interagiert,
(3) Bereitstellen einer Cas-Nuklease vom Typ II,
(4) In Kontakt bringen des doppelsträngigen zirkulären DNA-Moleküls mit der Führungs-RNA und der CAS-Nuklease.

Für das erfindungsgemäße Verfahren wird ein spezifisch gestaltetes, doppelsträngiges, zirkuläres DNA-Molekül bereitgestellt. Das zirkuläre DNA-Molekül umfasst auf dem Matrizen-Strang in der 3'- zu 5'-Richtung eine Templat-Sequenz, die operativ mit einem RNA-Polymerase-Promotor verbunden ist, eine Poly T-Sequenz, gefolgt von einer Nukleotidsequenz, dargestellt durch N₁N₂N₃, worin N₁ bis N₃ jeweils unabhängig voneinander G, T, A oder C oder ein Nukleotidanalogon davon darstellen, und ein *Protospacer Adjacent Motif* (PAM).

Der RNA-Polymerase-Promotor ist bevorzugt eine Erkennungssequenz für eine DNA-abhängige RNA-Polymerase, beispielsweise der T7 RNA-Polymerase-Promotor, der SP6 RNA-Polymerase-Promotor oder der T3 RNA-Polymerase-Promotor.

Die Templat-Sequenz umfasst beispielsweise eine Nukleinsäure, welche für ein Antigen, beispielsweise ein Tumorantigen, ein virales oder ein bakterielles Antigen kodiert. In einem anderen Beispiel umfasst die Templat-Sequenz eine Nukleinsäure, welche für ein therapeutisches Protein oder für ein anderes Protein wie z.B. einen Wachstumsfaktor oder Transkriptionsfaktor kodiert.

Die Poly T-Sequenz auf dem Matrizen-Strang des doppelsträngigen zirkulären DNA-Moleküls besteht beispielsweise aus zwischen etwa 40 bis etwa 250 T-Nukleotiden. Bevorzugt besteht die Poly T-Sequenz aus zwischen etwa 100 und etwa 140 T-Nukleotiden, und mehr bevorzugt aus etwa 120 T-Nukleotiden.

In der Nukleotidsequenz, die dargestellt ist durch N₁N₂N₃, stellen N₁ bis N₃ jeweils unabhängig voneinander G, T, A oder C oder ein Nukleotidanalogon davon dar. Bevorzugt stellen N₁ bis N₃ jeweils unabhängig voneinander G, T, A oder C dar. In einer besonders bevorzugten Ausführungsform stellen N₁ bis N₃ jeweils unabhängig voneinander G, A, oder C dar.

Auf dem Matrizen-Strang des doppelsträngigen zirkulären DNA-Moleküls befindet sich zudem ein *Protospacer Adjacent Motif* (PAM). Bevorzugt folgt das PAM unmittelbar auf die Nukleotidsequenz N₁N₂N₃.

Das PAM kann ein beliebig ausgewähltes PAM sein, in Abhängigkeit von der spezifischen Cas-Nuklease, welche im erfindungsgemäßen Verfahren eingesetzt werden soll. Beispielsweise umfasst das PAM eine Sequenz, welche von einer Cas-Nuklease des Typs II erkannt wird. Beispielsweise ist das PAM ausgewählt aus NGG, NGGNG, NNAAAAW, NG, NNNNACA, NNNNGATT, GNNNCNNA und NNAGNN, worin N jeweils unabhängig ausgewählt ist aus G, C, A und T, und wobei die PAM-Sequenzen in 5'-3'-Richtung dargestellt sind. In einer bevorzugten Ausführungsform umfasst das PAM die Sequenz 5'-NGG-3', worin N unabhängig ausgewählt ist aus G, C, A und T. In einer anderen bevorzugten Ausführungsform weist das PAM die Sequenz 5'-NGG-3' auf, wobei N unabhängig ausgewählt ist aus G, C, A oder T. In einer weiteren bevorzugten Ausführungsform wird das PAM von einer Cas9-Nuklease aus *Streptococcus* erkannt.

Die Zielstelle, an der die zirkuläre doppelsträngige DNA geschnitten werden soll, liegt bevorzugt 3 Nukleotide 5' vom PAM.

Die Führungs-RNA für das erfindungsgemäße Verfahren ist so gestaltet, dass sie am 5'-Ende eine Sequenz aufweist, welche komplementär zum Matrizen-Strang des doppelsträngigen zirkulären DNA-Moleküls ist. Diese Komplementaritätsregion besteht beispielsweise aus etwa 20 Nukleotiden, die komplementär sind zu etwa 20 aufeinander folgenden Nukleotiden des Matrizen-Strangs.

Die Führungs-RNA umfasst eine Region, die komplementär ist zum 5'-Ende der Poly T-Sequenz (ii) und zur Nukleotidsequenz (iii). Bevorzugt weist die Region der Führungs-RNA, die komplementär ist zum 5'-Ende der Poly T-Sequenz (ii)und zur Nukleotidsequenz (iii) die Sequenz 5'-G(A)ₙ-N₄N₅N₆-3' auf. Dabei ist n eine Zahl zwischen 17 und 23, bevorzugt eine Zahl zwischen 17 und 20 und mehr bevorzugt ist n gleich 17.

N₄ bis N₆ stellen jeweils unabhängig G, U, A, C oder ein Nukleotidanalogon davon dar, bevorzugt stellen N₄ bis N₆ jeweils unabhängig G, U, C oder ein Nukleotidanalogon dar, besonders bevorzugt stellen N₄ bis N₆ jeweils unabhängig G, U oder C dar.

Die Komplementaritätssequenz innerhalb der Führungs-RNA weist beispielsweise die Sequenz 5'-GAAAAAAAAAAAAAAAAANNN-3' auf, worin N jeweils unabhängig ein beliebiges Nukleotid ist. Bevorzugt ist das Verfahren dadurch gekennzeichnet, dass es sich bei der Führungs-RNA um SEQ ID NO:1 handelt.

Die Komplementarität der Region der Führungs-RNA zum 5'-Ende der Poly T-Sequenz (ii) und zur Nukleotidsequenz (iii) beträgt mindestens 90%, beispielsweise 90%, 91%, 92%, 93%, 94%, 95%, 96%, 97%, 98%, 99%, oder 100%. Bevorzugt beträgt die Komplementarität 98%, 99% oder 100%, besonders bevorzugt 100%.

Die Nukleotide N₄N₅N₆ der Führungs-RNA sind komplementär zu den Nukleotiden N₁N₂N₃ aus der Nukleotidsequenz (iii) aus dem Matrizen-Strang. Das bedeutet, dass N₄ komplementär ist zu N₁, N₅ komplementär ist zu N₂, und N₆ komplementär ist zu N₃. Bevorzugt beträgt die Komplementarität der Nukleotide N₄N₅N₆ und der Nukleotide N₁N₂N₃ 100%. Bevorzugte komplementäre Basenpaarungen umfassen beispielsweise G und C, A und T bzw. A und U. Komplementäre Sequenzen sind beispielsweise CAG für N₁N₂N₃ und GUC für N₄N₅N₆.

Weiter umfasst die Führungs-RNA eine Region, die mit einer Cas-Nuklease interagiert. Bevorzugt umfasst die Führungs-RNA eine Region, die mit einer Cas-Nuklease vom Typ II interagiert.

Weiter wird in dem erfindungsgemäßen Verfahren eine Cas-Nuklease vom Typ II bereitgestellt. Es kann hierbei eine beliebige Cas-Nuklease vom Typ II eingesetzt werden. Beispielsweise wird eine Cas Nuklease vom Typ II aus *Streptococcus pyogenes* (SpCas9; Uniprot Q99ZW2), *Streptococcus mutans* (SmCas9; Uniprot Q8DTE3), *Streptococcus thermophilus* (StCas9; Unitprot G3ECR1), *Staphylococcus aureus* (SaCas9; Uniprot J7RUA5), *Francisella novicida* (FnCas9; Uniprot A0Q5Y3), *Campylobacter jejuni* (CjCas9; Uniprot A0A698TVI8), *Neisseria meningitids* (MnCas9; Uniprot C9X1G5), *Pasteurella multocida* (PmCas9; AddGene#68703; https://www.addgene.org/68703/) bereitgestellt. Bevorzugt wird eine Cas-Nuklease vom Typ II aus *Streptococcus spec.* bereitgestellt. Mehr bevorzugt wird eine Cas-Nuklease vom Typ II aus *Streptococcus pyogenes* bereitgestellt. Am meisten bevorzugt wird als Cas-Nuklease vom Typ II Cas9 aus *Streptococcus pyogenes* eingesetzt.

Bevorzugt wird als Cas-Nuklease vom Typ II Cas9 eingesetzt. Beispielsweise wird Cas9 ausgewählt aus *Streptococcus pyogenes* mit der SEQ ID NO:2 oder einer dazu zu mindestens 70% homologen Sequenz, bevorzugt mindestens 80% homologen Sequenz, mehr bevorzugt mindestens 90% homologen Sequenz, mehr bevorzugt mindestens 95% homologen Sequenz und besonders bevorzugt mindestens 98% homologen Sequenz; *Staphylococcus aureus* mit der SEQ ID NO:3 oder einer dazu zu mindestens 70% homologen Sequenz, bevorzugt mindestens 80% homologen Sequenz, mehr bevorzugt mindestens 90% homologen Sequenz, mehr bevorzugt mindestens 95% homologen Sequenz und besonders bevorzugt mindestens 98% homologen Sequenz, oder *Streptococcus thermophilus* mit der SEQ ID NO:4 oder einer dazu zu mindestens 70% homologen Sequenz, bevorzugt mindestens 80% homologen Sequenz, mehr bevorzugt mindestens 90% homologen Sequenz, mehr bevorzugt mindestens 95% homologen Sequenz und besonders bevorzugt mindestens 98% homologen Sequenz.

In einer bevorzugten Ausführungsform handelt es sich bei der Cas-Nuklease vom Typ II um Cas9 aus *Streptococcus pyogenes* mit der SEQ ID NO:2 oder einer dazu zu mindestens 70% homologen Sequenz, bevorzugt mindestens 80% homologen Sequenz, mehr bevorzugt mindestens 90% homologen Sequenz, mehr bevorzugt mindestens 95% homologen Sequenz und besonders bevorzugt mindestens 98% homologen Sequenz.

Wenn im erfindungsgemäßen Verfahren als Cas-Nuklease Cas9 aus *S. pyogenes* eingesetzt wird, dann wird als PAM bevorzugt 5'-NGG-3' verwendet. Wenn als Cas-Nuklease Cas9 aus *S. mutans* eingesetzt wird, dann wird als PAM bevorzugt 5'-NGG-3' verwendet. Wenn als Cas-Nuklease eine Cas9 Variante aus *S. thermophilus* eingesetzt wird, dann wird als PAM bevorzugt 5'-NGGNG-3' oder 5'-NNAAAAW-3' verwendet. Wenn als Cas-Nuklease Cas9 aus *Francisella novicida* eingesetzt wird, dann wird als PAM bevorzugt 5'-NG-3' verwendet. Wenn als Cas-Nuklease Cas9 aus *Campylobacter jejuni* eingesetzt wird, dann wird als PAM bevorzugt 5'-NNNNACA-3' verwendet. Wenn als Cas-Nuklease Cas9 aus *Neisseria menigitidis* eingesetzt wird, dann wird als PAM bevorzugt 5'-NNNNGATT-3' verwendet. Wenn als Cas-Nuklease Cas9 aus *Pasteurella multocida* eingesetzt wird, dann wird als PAM bevorzugt 5'-GNNNCNNA-3', verwendet, wobei die vorliegende Erfindung nicht auf diese Beispiele beschränkt sein soll.

In einer weiteren bevorzugten Ausführungsform betrifft die vorliegende Erfindung ein Verfahren zur Herstellung eines linearen doppelsträngigen DNA-Moleküls, worin das lineare DNA-Molekül glatte Enden aufweist, und worin das lineare DNA-Molekül eine Poly T-Sequenz am 5' Ende des Matrizen-Strangs aufweist, umfassend die Schritte:
(1) Bereitstellen eines doppelsträngigen zirkulären DNA-Moleküls, umfassend auf dem Matrizen-Strang in der 3'- zu 5-Richtung:
   (i) eine Templat-Sequenz, die operativ mit einem RNA-Polymerase-Promotor verbunden ist,
   (ii) eine Poly T-Sequenz, worin die Poly T-Sequenz aus etwa 120 T-Nukleotiden besteht,
   (iii) eine Nukleotidsequenz, dargestellt durch N₁N₂N₃, worin N₁ bis N₃ jeweils unabhängig voneinander G, T, A oder C oder ein Nukleotidanalogon davon darstellen, und
   (iv) ein PAM, welches die Sequenz 5'-NGG-3' aufweist, wobei N jeweils unabhängig ausgewählt ist aus G, C, A und T,
(2) Bereitstellen einer Führungs-RNA, umfassend eine Region, die komplementär ist zum 5'-Ende der Poly T-Sequenz (ii) und zur Nukleotidsequenz (iii), und eine Region, die mit einer Cas-Nuklease interagiert, worin die Region, die komplementär ist zum 5'-Ende der Poly T-Sequenz (ii)und zur Nukleotidsequenz (iii) die Sequenz 5'-G(A)ₙN₄N₅N₆-3' aufweist, worin n gleich 17 ist, worin N₄ bis N₆ jeweils unabhängig G, U oder C darstellt,
(3) Bereitstellen einer Cas-Nuklease vom Typ II, worin die Cas-Nuklease vom Typ II Cas9 aus *Streptococcus pyogenes* mit der SEQ ID NO:2 oder einer dazu zu mindestens 70% homologen Sequenz ist,
(4) In Kontakt bringen des doppelsträngigen zirkulären DNA-Moleküls mit der Führungs-RNA und der CAS-Nuklease.

Die Führungs-RNA kann im erfindungsgemäßen Verfahren als RNA-Molekül bereitgestellt werden. Das Bereitstellen der Führungs-RNA kann erfindungsgemäß auch durch die Expression einer Nukleotidsequenz erfolgen, welche die Führungs-RNA kodiert. Bevorzugt wird die Führungs-RNA durch die Expression einer Nukleotidsequenz bereitgestellt, welche die Führungs-RNA kodiert.

Die Cas-Nuklease vom Typ II kann im erfindungsgemäßen Verfahren als Protein bereitgestellt werden. Das Bereitstellen der Cas-Nuklease vom Typ II kann erfindungsgemäß auch durch die Expression einer Nukleotidsequenz erfolgen, welche für die Cas-Nuklease vom Typ II kodiert. Bevorzugt wird die Cas-Nuklease vom Typ II durch die Expression einer Nukleotidsequenz bereitgestellt, welche die Cas-Nuklease vom Typ II kodiert. Besonders bevorzugt werden sowohl die Führungs-RNA als auch die Cas-Nuklease durch die Expression einer Nukleotidsequenz bereitgestellt. Die Nukleinsäure, welche die Führungs-RNA und/oder die Cas-Nuklease kodiert, kann auf einem Expressionsplasmid lokalisiert sein. Beispielsweise kann die Nukleinsäure, welche die Führungs-RNA kodiert, auf einem Expressionsplasmid lokalisiert sein, und die Nukleinsäure, welche die die Cas-Nuklease kodiert, kann auf einem separaten Expressionsplasmid lokalisiert sein. In einem anderen Beispiel sind die Nukleinsäure, welche die Führungs-RNA kodiert und die Nukleinsäure, welche die Cas-Nuklease kodiert, auf demselben Expressionsplasmid lokalisiert.

Beispielsweise kann ein Expressionsplasmid mit Cas9 und Führungs-RNA wie folgt generiert werden. Bei der Klonierung kann zunächst eine Cas9-Nuklease in ein geeignetes Expressionsplasmid kloniert werden, und anschließend kann beispielsweise in 5' - 3' Richtung ein tracrRNA Konstrukt und ein minimaler CRISPR Array kloniert werden. Dies ermöglicht beispielsweise eine natürliche Formierung einer tracrRNA::crRNA Duplex einer Führungs-RNA. Ein geeigneter Expressionsvektor ist beispielsweise pWAC003 (SEQ ID NO:5). Dabei handelt es sich um ein *low-copy* Plasmid mit ca. 10 Kopien pro Zelle auf Basis des pACYC-Replikationsursprungs.

Erfindungsgemäß erfolgt ein in Kontakt bringen des doppelsträngigen zirkulären DNA-Moleküls mit der Führungs-RNA und der Cas-Nuklease. Das in Kontakt bringen kann beispielsweise *in vitro* erfolgen. In einer Ausführungsform, worin das Bereitstellen der Führungs-RNA und/oder der Cas-Nuklease vom Typ II durch die Expression einer Nukleotidsequenz erfolgt, welche die Führungs-RNA und/oder die Cas-Nuklease vom Typ II kodiert, erfolgt das in Kontakt bringen bevorzugt in einer Zelle, insbesondere in einer Bakterienzelle, welche geeignet ist, Plasmid-DNA zu vermehren.

Eine konstitutive Expression der Cas-Endonuklease, beispielsweise Cas9, kann unspezifische DNA-Schnittereignisse an verschiedenen Stellen im Plasmid-Produkt, d.h. im doppelsträngigen zirkulären DNA-Molekül, oder im Chromosom der verwendeten Bakterienzelle verursachen. Ein frühes Prozessieren des doppelsträngigen zirkulären DNA-Moleküls durch eine Cas-Nuklease, beispielsweise Cas9, in den Zellen kann so zum Abbau des gewünschten linearen DNA-Produkts führen, da die freien Enden einer linearen DNA von entsprechenden DNasen erkannt werden. Zudem kann auch die Zelle als Produzent geschädigt werden. Um diesen Effekt zu verringern ist es bevorzugt, für die Expression der Cas-Nuklease vom Typ II, beispielsweise Cas9, einen regulierbaren Promotor zu verwenden, der eine möglichst geringe Basalexpression aufweist.

Für die induzierbare Expression der Cas-Nuklease, beispielsweise Cas9, ist zusätzlich dazu eine mögliche Begrenzung der Promoter-Derepression, d.h. der induzierbaren Expressionshöhe bevorzugt. Eine zu hohe Expression der Cas-Nuklease, beispielsweise Cas9, in einer kurzen Zeitspanne, kann ähnlich negative Effekte auf die pDNA-Qualität und die Produzentenzelle haben, wie eine hohe Basalexpression über eine längere Zeitspanne. Daher sind solche Promotoren besonders bevorzugt, die eine geringe Basalexpression und nach Induktion eine moderate Expressionsstärke aufweisen.

Eine induzierbare Expression der Führungs-RNA hat gegenüber einer konstitutiven Expression den Vorteil, dass die Expression keine dauerhafte Belastung für die Produzentenzelle darstellt. Durch den geringeren Stress kann die Zelle mehr vom eigentlichen Zielprodukt, der zirkulären DNA herstellen.

Durch die Verwendung eines regulierbaren bzw. induzierbaren Promotors kann der Zeitpunkt der Produktion der Cas-Nuklease und der Führungs-RNA gezielt durch die Induktion der Expression gewählt werden. Durch eine regulierbare bzw. induzierbare Expression des Prozessierungsapparates, d.h. der Cas9-Nuklease und der Führungs-RNA, innerhalb der Zelle, können im Folgeprozess einzelne Schritte eingespart werden, was zu wirtschaftlichen Vorteilen führt. Beispielsweise können zusätzliche Linearisierungsschritte oder eine separate Aufreinigung der Plasmid-DNA entfallen.

Bevorzugt ist das erfindungsgemäße Verfahren dadurch gekennzeichnet, dass die Expression der Cas-Nuklease vom Typ II und/oder der Führungs-RNA induzierbar ist. Das bedeutet, dass die Nukleotidsequenz kodierend für die Führungs-RNA und/oder die Nukleotidsequenz kodierend für die Cas-Nuklease operativ mit einem induzierbaren Promotor verbunden ist. Bevorzugt ist sowohl die Nukleotidsequenz kodierend für die Führungs-RNA als auch die Nukleotidsequenz kodierend für die Cas-Nuklease operativ mit einem induzierbaren Promotor verbunden.

In einer Ausführungsform ist die Nukleotidsequenz kodierend für die Führungs-RNA und die Nukleotidsequenz kodierend für die Cas-Nuklease operativ mit dem gleichen induzierbaren Promotor verbunden. Bei Verwendung eines gleichen oder ähnlichen Induktionssystems beginnt die Expression der Führungs-RNA und der Cas-Nuklease gleichzeitig. Zum einen erlaubt dieses Vorgehen die Senkung des zellulären Stresses, andererseits wird durch den zeitlich abgestimmten Beginn der Expression, ein passenderes Verhältnis der Moleküle Cas-Endonuklease vs. Führungs-RNA ermöglicht.

In einer anderen Ausführungsform ist die Nukleotidsequenz kodierend für die Führungs-RNA und die Nukleotidsequenz kodierend für die Cas-Nuklease operativ mit unterschiedlichen induzierbaren Promotoren, z.B. für sequentielle Expressionen, verbunden.

Induzierbare Promotoren sind sind einem Fachmann bekannt. Als induzierbare Promotoren eignen sich beispielsweise der lac-, tac-, trc-, lambda PL, ara-, cumat- oder tet-Promotor oder davon abgeleitete Sequenzen.

Bevorzugt wird erfindungsgemäß als induzierbarer Promotor ein durch Lactose-induzierbarer Promotor verwendet, insbesondere der Promotor WAC003 mit der Sequenz SEQ ID NO:6 oder der Promotor WAC003-LacIDel mit der Sequenz SEQ ID NO:7.

In einer Ausführungsform ist die Nukleotidsequenz kodierend für die Führungs-RNA und/oder die Nukleotidsequenz kodierend für die Cas-Nuklease in das Genom von *E. coli* integriert. In einer bevorzugten Ausführungsform ist sowohl die Nukleotidsequenz kodierend für die Führungs-RNA als auch die Nukleotidsequenz kodierend für die Cas-Nuklease in das Genom von *E*. *coli* integriert. In einer anderen bevorzugten Ausführungsform ist die Nukleotidsequenz kodierend für die Cas-Nuklease in das Genom von *E. coli* integriert, nicht aber die Nukleotidsequenz kodierend für die Führungs-RNA. Verfahren zum Integrieren von Nukleotidsequenzen in bakterielle Genome sind einem Fachmann bekannt (z.B. Datsenko et al. 2000, Proc. Natl. Acad. Sci. U S A. 97: 6640 - 5).

In einem anderen Aspekt betrifft die vorliegende Erfindung die Verwendung einer Cas-Nuklease vom Typ II zur Linearisierung eines doppelsträngigen, zirkulären DNA-Moleküls, worin das lineare DNA-Molekül auf dem Matrizen-Strang in der 3'- zu 5'-Richtung umfasst:
(i) eine Templat-Sequenz, die operativ mit einem RNA-Polymerase-Promotor verbunden ist,
(ii) eine Poly T-Sequenz,
(iii) eine Nukleotidsequenz, dargestellt durch N₁N₂N₃, worin N₁ bis N₃ jeweils unabhängig voneinander G, T, A oder C oder ein Nukleotidanalogon davon darstellen, und
(iv) ein Protospacer Adjacent Motif (PAM).

In einem weiteren Aspekt betrifft die vorliegende Erfindung die Verwendung eines rekombinanten Mikroorganismus, worin die Nukleotidsequenz kodierend für eine Führungs-RNA und/oder die Nukleotidsequenz kodierend für eine Cas-Nuklease vom Typ II in das Genom integriert ist, zur Herstellung eines linearen doppelsträngigen DNA-Moleküls, worin das lineare DNA-Molekül glatte Enden aufweist, und worin das lineare DNA-Molekül eine Poly T-Sequenz am 5' Ende des Matrizen-Stranges aufweist.

Eine Ausführungsform betrifft die Verwendung eines rekombinanten Mikroorganismus, worin die Nukleotidsequenz kodierend für eine Führungs-RNA und die Nukleotidsequenz kodierend für eine Cas-Nuklease vom Typ II in das Genom integriert ist. Eine andere Ausführungsform betrifft die Verwendung eines rekombinanten Mikroorganismus, worin die Nukleotidsequenz kodierend für eine Cas-Nuklease vom Typ II in das Genom integriert ist, zur Herstellung eines linearen doppelsträngigen DNA-Moleküls.

Der rekombinante Mikroorganismus kann ein beliebiger, für die Erzeugung von Plasmid-DNA geeigneter Mikroorganismus sein. Bevorzugt ist der rekombinante Mikroorganismus *E. coli,* mehr bevorzugt ist der rekombinante Mikroorganismus ein *E. coli* K12 Stamm, besonders bevorzugt ist der rekombinante Mikroorganismus *E. coli* NEB10Beta.

Die Erfindung wird weiterhin im Folgenden anhand von Ausführungsbeispielen und den beigefügten Abbildungen näher beschrieben, ohne dadurch beschränkt zu werden.

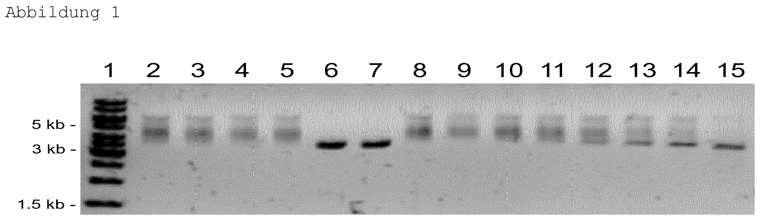

Abbildung 1 zeigt ein Agarose-Gel mit einer gelelektrophoretisch aufgetrennten Plasmid-DNA und verschiedenen Kontrollen: 1: Marker (GeneRuler^{™} 1 kb DNA Ladder) 2: pGFP1 ohne Inkubation (Negativkontrolle 1) 3: pGFP1 in Wasser mit Inkubation (Negativkontrolle 2) 4: pGFP1 in Schneidepuffer (Negativkontrolle 3) 5: pGFP1 in Schneidepuffer + 1 mM EDTA (Negativkontrolle 4) 6: pGFP1 inkubiert mit XbaI (Positivkontrolle 1) 7: pGFP1 inkubiert mit NcoI (Postivkontrolle 2) 8: pGFP1 inkubiert mit 25 nM Cas9 (Negativkontrolle 5) 9: pGFP1 inkubiert mit 25 nM sgRNA (Negativkontrolle 6) 10-15: Testproben 1-5 (1-facher, 2-facher, 3-facher, 4-facher, 5-facher und 10-facher Überschuss Cas9/Führungs-RNA zu pDNA).

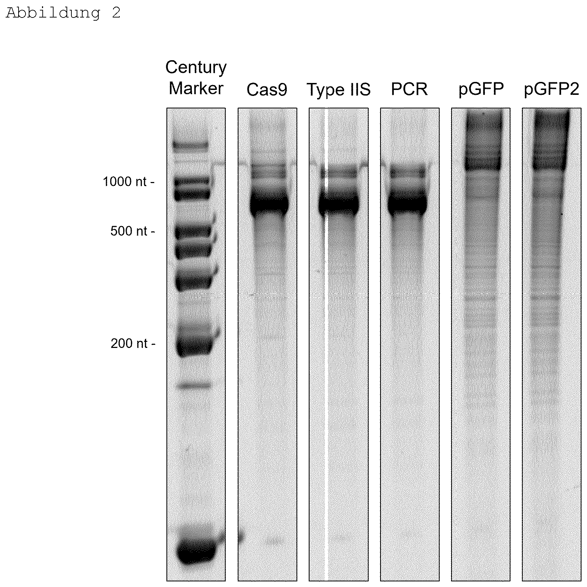

Abbildung 2 zeigt ein Agarosegel mit gelelektrophoretisch aufgetrennten mRNAs, die in *in* vitro-Transkriptionen unter Verwendung unterschiedlicher DNA-Template gebildet wurden. Cas9 bezeichnet die Probe, worin die mittels SpCas9/Führungs-RNA linearisierte DNA als Templat-DNA verwendet wurde. Typ IIS bezeichnet die Probe, worin die mittels Typ IIS Restriktionsenzym linearisierte DNA als Template-DNA verwendet wurde. PCR bezeichnet die Probe, worin die mittels PCR erzeugte DNA als Templat verwendet wurde. pGFP und pGFP2 bezeichnen die Proben, worin die zirkulären Plasmide pGFP1 und pGFP2 als DNA-Template verwendet wurden.

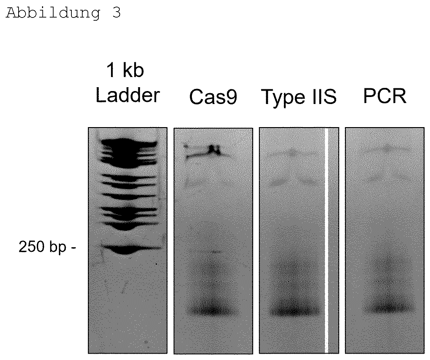

Abbildung 3 zeigt die Analyse des Poly A-Anhangs mittels Acrylamid-Gelelektrophorese. 1: Marker, GeneRuler^{™} 1 kb DNA Ladder (Thermo Fisher Scientifc). Cas9: RT-PCR-Produkt für die RNA, welche mit einer mittels SpCas9-linearisierten Templat-DNA hergestellt wurde. TypIIS: RT-PCR-Produkt für die RNA, welche mit einer mittels Typ IIS Restriktionsenzym linearisierten Templat-DNA hergestellt wurde. PCR: RT-PCR-Produkt für die RNA, welche mit einer mittels PCR hergestellten Templat-DNA hergestellt wurde.

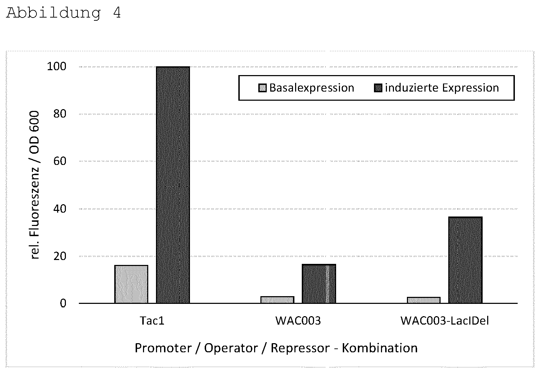

Abbildung 4 zeigt die Basalexpression und Expressionsstärke der veränderten Promotoren WAC003 und WAC003-LacIDel im Vergleich zur pTac1 Ausgangsvariante am Beispiel der Expression des Reporterproteins GFP in *E.coli* K12.

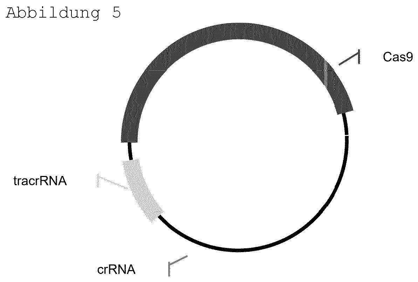

Abbildung 5 zeigt eine Schematische Darstellung des Plasmids pCas9_tracrRNA_CRISPR.

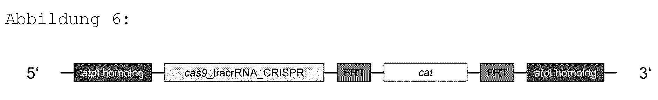

Abbildung 6 ist eine schematische Darstellung eines Konstrukts für die genomische Integration mittels λ-Red Rekombination.

### Beispiele

### Beispiel 1: Plasmid-DNA-Schneideaktivität durch Cas9

Zunächst wurde für die Herstellung des Plasmids ein Konstrukt mit Templat-Sequenz, Poly A-Anhang sowie Zielsequenz für den Schnitt einer Cas-Nuklease und PAM entworfen. Dieses Konstrukt kodiert neben einer GFP-mRNA Templat-Sequenz am 3' Ende dieser Sequenz für einen Poly A-Anhang, welcher wiederum von drei weiteren Nukleotiden (5'-GTC-3') sowie dem *Protospacer Adjacent Motif* (PAM) 5'-CGG-3' gefolgt wird. Die PAM-Sequenz stellt die Cas9-gRNA Erkennungssequenz zur Prozessierung dar. Für die Erstellung der Templat-DNA Sequenz wurde das gfp-Gen (Uniprot: C5MKY7) mit den beiden dafür entsprechenden Primern (SEQ ID NO:8 und 9) in einer PCR nach Standardprotokoll mit der Phusion Polymerase amplifiziert (Thermo Fisher Scientific). Die beiden Primer umfassten zum einen den T7-RNA-Polymerase-Promotor (Forward Primer, SEQ ID NO:8) und zum anderen die Elemente: Poly T, 5' GAC 3' und PAM (5'-CGG-3') (Reverse Primer, SEQ ID NO:9). Das PCR-Produkt (SEQ ID Nr:10) wurde anschließend über die *XbaI-* und EcoRI-Schnittstellen in einen pUC18-Vektor (New England Biolabs) kloniert. Das resultierende Plasmid pGFP1 (SEQ ID NO:11) wurde in den folgenden Versuchen/Beispielen als Ausgangsplasmid für die Herstellung der linearisierten Templat-DNA eingesetzt.

Nach erfolgreicher Herstellung des Plasmids pGFP1 wurde die Plasmid-DNA mit Hilfe von *E. coli* NEB10Beta (New England Biolabs) produziert. Dazu wurden die NEB10Beta Zellen nach Herstellerangaben mittels Hitzeschock-Methode mit dem Plasmid pGFP1 transformiert. Hierfür wurden die Zellen und 100 ng pGFP1 für 30 min auf Eis inkubiert, bevor ein 30 Sekunden Hitzeschock bei 42 °C erfolgte. Nach einer Regenerationsphase (1 h bei 37 °C) folgte die Selektion der Transformanden auf Selektionsmedium (LB-Ampicillin (LB-Amp)). Entstandene Klone wurden in 3 mL-Kulturen unter selektiven Bedingungen angezogen und das Plasmid der Klone über Restriktionsmuster und Sequenzierung überprüft. Klone mit dem gewünschten Plasmid wurden in 50 mL LB-Amp angezogen (schüttelnd, 37 °C über Nacht), um ausreichende Mengen an Plasmid-DNA zu generieren. Je nach Klon wurde eine OD₆₀₀ (optische Dichte bei 600nm gegen Wasser) von 8-14 im Schüttelkolben erreicht. Die Plasmid-DNA wurde abschließend mit dem Gene Jet Plasmid Kit (Thermo Fisher Scientific) entsprechend den Angaben des Herstellers aus der Über-Nacht-Kultur (stationäre Phase wurde erreicht) isoliert und gereinigt.

In einem nächsten Schritt wurde eine Templat-DNA für die *in vitro-*Herstellung einer Führungs-RNA (SEQ ID NO:1) entworfen. Um die Synthese einer Führungs-RNA mit spezifischer Länge und spezifischer Sequenz zu ermöglichen, wurde die DNA-Sequenz komplementär zu der Führungs-RNA 3' hinter eine T7 RNA-Polymerase-Promotorsequenz angefügt (SEQ ID NO:12). Zusammen mit der komplementären Sequenz des T7 Promoters (SEQ ID NO:13) wurde dieses als lange Einzelstränge (Primer) synthetisiert (Fa. Metabion).

Zur Herstellung der Führungs-RNA (SEQ ID NO:1) wurden diese beiden DNA-Oligonukleotide (SEQ ID NO:12 und 13) miteinander hybridisiert, indem beide in äquimolaren Mengen (100 pmol) gemischt, für 5 min bei 95 °C aufgekocht und anschließend langsam auf Raumtemperatur heruntergekühlt wurden. Das erzeugte Hybrid wurde anschließend als Matrize genutzt, um die Führungs-RNA mit einem *in* vitro-Transkriptionskit (HighYield T7 RNA Synthesis Kit von Jena Bioscience) nach Herstellerangaben zu synthetisieren. Anschließend wurde die Transkriptionsreaktion mit dem Monarch RNA Cleanup Kit von New England Biolabs (NEB) nach Herstellerangaben gereinigt und die Konzentration mittels UV-Absorption bestimmt.

Nach der Herstellung des Plasmids pGFP1 (SEQ ID NO:11) und der Führungs-RNA (SEQ ID NO:1), wurden beide zusammen mit der Cas-Nuklease SpCas9 (New England Biolabs) in einem Schneidepuffer inkubiert (20 mM Hepes pH 7.5; 150 mM KCl, 10 mM MgCl₂, 0.5 mM DTT, 0.1 mM EDTA). Dabei wurden SpCas9 und die Führungs-RNA äquimolar eingesetzt und jeweils in verschiedenen Überschüssen zur pDNA (5 nM) (1-fach, 2-fach, 3-fach, 4-fach, 5-fach und 10-fach) verwendet. Zur besseren Komplexbildung, wurden SpCas9 und die Führungs-RNA zunächst allein inkubiert (15 min, 37 °C). Anschließend wurden die pDNA (5 nM) und der Reaktionspuffer hinzugefügt. Nach 60 min Inkubationszeit bei 37 °C wurde die Reaktion mit Zugabe von 20 µL 2x Formamid-Puffer (Thermo Fisher Scientific) abgestoppt und die pDNA mittels Agarose-Gelelektrophorese analysiert (Abbildung 1).

Für die Analyse wurde die geschnittene pDNA über ein 1%-iges Agarose-Gel (TAE gepuffert) getrennt und anschließend mittels Midori Green (Nippon Genetics Europe) an einer Geldokumentationsanalage (BioRad) visualisiert (siehe Abbildung 1). Als Kontrollen dienten ungeschnittene und nicht inkubierte Plasmid-DNA, ungeschnittene inkubierte (1h, 37 °C) pDNA, pDNA mit Schneidepuffer, pDNA mit Schneidepuffer und 1 mM EDTA, pDNA mit 25 nM Cas9 ohne Führungs-RNA, pDNA mit 25 nM Führungs-RNA ohne Cas9, sowie als Positivkontrolle pDNA (SEQ ID NO:11), die mittels *XbaI* bzw. NcoI (verwendet nach Herstellerangaben, Thermo Fisher Scientific) linearisiert wurde.

Abbildung 1 zeigt, dass die Plasmid-DNA pGFP1 durch den Cas9/Führungs-RNA Komplex linearisiert wird. Aufgetragen wurde in Spur 1 der Marker, GeneRuler^{™} 1 kb DNA Ladder (Thermo Fisher Scientifc). Als Kontrollen dienten: pGFP1 ohne Inkubation (Spur 2, Ausgangsmaterial), pGFP1 in Wasser mit Inkubation (Spur 3, ohne Cas9 und Führungs-RNA), pGFP1 in Schneidepuffer (Spur 4, ohne Cas9 und Führungs-RNA), pGFP1 in Schneidepuffer + 1 mM EDTA (Spur 5, ohne Cas9 und Führungs-RNA), pGFP1 inkubiert mit XbaI (Spur 6), pGFP1 inkubiert mit NcoI (Spur 7), pGFP1 inkubiert mit 25 nM Cas9 (ohne Führungs-RNA; Spur 8), pGFP1 inkubiert mit 25 nM Führungs-RNA (ohne Cas9; Spur 9). Die Testproben wurden in den Spuren 10 - 15 aufgetragen (1-fach, 2-facher, 3-facher, 4-facher, 5-facher und 10-facher Überschuss Cas9/Führungs-RNA zu pDNA). Abbildung 1 zeigt zudem, dass bei zunehmender Menge des Cas9/ Führungs-RNA-Komplexes mehr pDNA linearisiert wird. Bei einem 10-fachen molaren Überschuss (Spur 15) ist nahezu der gleiche Grad der Linearisierung erreicht, wie bei der Verwendung von herkömmlichen Type II-Restriktionsenzymen. Dies ist exemplarisch mit den Reaktionen unter Nutzung von *XbaI* und NcoI (Spuren 6 & 7) dargestellt. Die Abhängigkeit von der Cas9/Führungs-RNA-Komplexbildung zeigen die beiden Spuren 8 und 9, in denen bei einem 10-fachen molaren Überschuss jeweils eine Komponente, entweder die Führungs-RNA (Spur 8) oder Cas9 (Spur 9), weggelassen wurde. Hier ist keine Linearisierung zu beobachten, ebenso wie bei pGFP1 Proben, in denen keine Enzyme verwendet wurden (Spur 3, Spur 4, Spur 5).

### Beispiel 2: mRNA-Synthese aus pDNA, welche durch Cas9 geschnitten wurde

Die Plasmid-DNA pGFP1, (SEQ ID NO:11) wurde wie in Beispiel 1 beschrieben mittels SpCas9 und der Führungs-RNA (SEQ ID NO:1) linearisiert (10-facher molarer Überschuss, siehe auch Abbildung 1, Spur 15). Das linearisierte Plasmid wurde anschließend über das Gene Jet PCR Purification Kit (Thermo Fisher Scientific) gereinigt. Die lineare, aufgereinigte pDNA wurde anschließend als Templat-DNA in einer *in* vitro-Transkriptionsreaktion (HighYield T7 RNA Synthesis Kit, Jena Bioscience) nach Herstellerangaben eingesetzt, um eine mRNA zu synthetisieren. Die Reaktion wurde für 2 h bei 37 °C durchgeführt, dabei wurden 200 ng Templat-DNA eingesetzt. Um Verunreinigungen und Kitbestandteile, wie beispielsweise Enzyme und Nukleotide, aus der Reaktion zu entfernen, wurde die mRNA entsprechend den Herstellerangaben über ein Monarch RNA Cleanup Kit (NEB) aufgereinigt.

Um mRNA-Herstellungsprozesse unter Verwendung des erfindungsgemäßen Verfahrens mit den Methoden aus dem Stand der Technik zu vergleichen, d.h. Linearisierung einer Plasmid-DNA mittels Typ IIS Restriktionsenzymen bzw. Herstellung eines PCR-Produktes als lineare Ausgangs-DNA, wurde pGFP2 (SEQ ID NO:14, hergestellt in Analogie zu pGFP1, durch Amplifikation des gfp Gens mittels Primern der SEQ ID NO:9 und 15.) mit dem Typ IIS Restriktionsenzym *BpiI* nach Herstellerangaben (Thermo Fisher Scientific) behandelt und linearisiert. Zusätzlich wurde eine lineare Ausgangs-DNA über eine Polymerase-Ketten-Reaktion (PCR-Produkt) (SEQ ID NO:16) hergestellt. Dabei wurde ein Primer mit einem T7 RNA-Polymerase-Promoter (Forward Primer, SEQ ID NO:17) zusammen mit einem Primer mit einem 60 nt Poly A-Anhang (Reverse Primer, SEQ ID NO:18) sowie mit pGFP1 als PCR-Vorlage (10 ng) und der Phusion DNA Polymerase verwendet. Die Reaktion wurde im mitgelieferten Puffersystem nach Herstellerangaben durchgeführt (Thermo Fisher Scientific). Das PCR-Produkt wurde nach der Reaktion mit *DpnI* verdaut, um verbliebene methylierte Plasmid-DNA aus der PCR-Reaktion zu entfernen. Abschließend wurden sowohl das PCR-Produkt als auch das Typ IIS linearisierte Plasmid über ein PCR Purification Kit (Thermo Fisher Scientific) nach Herstellerangaben gereinigt. Während die lineare DNA aus der PCR-Reaktion bzw. die mit SpCas9 linearisierte DNA jeweils glatte ("*blunt*") Enden aufweisen, weist die Type IIS linearisierte DNA einen 3' Überhang auf.

Die linearen DNAs aus dem Restriktionsverdau und der PCR-Reaktion wurden in den gleichen Mengen wie die mittels SpCas9 linearisierte pDNA (200 ng) nach Herstellerangaben in einer *in vitro-*Transkriptionsreaktion als Templat-DNA zur Synthese einer mRNA eingesetzt (HighYield T7 RNA Synthesis Kit, Jena Bioscience). Die Synthese wurde bei 37°C für 2h durchgeführt. Abschließend wurden die mRNAs über das Monarch RNA Cleanup Kit (NEB) entsprechend der Herstellerangaben gereinigt.

Die Analyse der hergestellten mRNAs erfolgte zunächst über eine Polyacrylamid-Gelelektrophorese. Dabei wurden 200 ng mRNA von jedem Ansatz mit RNA-Ladepuffer (Gel Loading Buffer 2, Thermo Fisher Scientific) versetzt und für 5 min bei 95 °C aufgekocht. Dann erfolgte die Analyse der RNA-Spezies über die Auftrennung auf einem 15 % TBE-UREA Polyacrylamid Gel (Thermo Fisher Scientific; 250 V, 50 min). Im Anschluss an die Elektrophorese wurde das Gel mittels SYBR Green II RNA Gel Stain (Thermo Fisher Scientific) gefärbt und in einer Geldokumentationsanlage (BioRad) visualisiert (Abbildung 2). Als Kontrollen dienten *in vitro-*Transkriptionsansätze mit 200 ng ungeschnittenem pGFP1 und pGFP2. pGFP2 ist eine pGFP1 Variante, welche keine PAM-Sequenz enthält (SEQ ID NO:14). Die Konzentration der hergestellten mRNAs wurde photometrisch mit einem NanoDrop-Photospektrometer (Thermo Fisher Scientific) bestimmt.

Abbildung 2 ist eine Visualisierung der hergestellten mRNAs. In Spur 1 befindet sich der Century Marker^{™} (Thermo Fisher Scientific) und in den folgenden Spuren die RNA-Proben entsprechend der Ausgangs-DNAs: SpCas9/Führungs-RNA linearisierte DNA (Cas9), mit Typ IIS Restriktionsenzym linearisierte DNA (Type IIS), DNA aus der PCR-Reaktion (PCR) sowie die ungeschnittenen Plasmide pGFP1 und pGFP2. Abbildung 2 zeigt deutlich, dass die erhaltenen mRNA-Hauptprodukte aus den Reaktionen mit linearisierten Plasmiden (Cas9, Type IIS) und PCR-Produkt (PCR) identische Größen aufweisen. Nur bei Reaktionen mit nicht linearisierten Plasmiden (pGFP1 und pGFP2) zeigen sich größere Produkte. Diese Produkte resultieren aus der "ungestoppten" *in* vitro-Transkription der T7 RNA-Polymerase. Durch die fehlende Linearisierung ist kein *"Run-OFF"* der Polymerase möglich was zu einem "Durchlesen" und damit zu heterogenen, größeren Produkten führt.

Für die Analyse des Poly A-Anhanges der mRNAs wurde mittels einer T4 RNA-Ligase 1 (NEB) ein Adapter-Oligonukleotid (SEQ ID NO:19) an das 3' Ende der mRNA ligiert. Die T4 RNA-Ligase 1 ist ein Enzym, welches einzelsträngige RNA ligieren kann. Dabei wird ein 5' Monophosphat-haltiges Substrat an ein 3' OH-Ende der Empfänger-mRNA ligiert. Das 3' OH-Ende enthält die hergestellte RNA natürlicherweise. Das Adapter-Oligonukleotid wurde so synthetisiert, dass es ein 5' Monophosphat enthält (Metabion). Um eine Selbstligation der Adapter-Oligonukleotide zu verhindern, wurde auch das 3' Ende des Adapter-Oligonukleotides mit einem synthetischen Phosphatrest versehen. Für die Ligation wurden 5 µg RNA und 40 pmol des Adapter-Oligonukleotids eingesetzt. Die Reaktion (50 mM Tris-HCl pH 7.5, 10 mM MgCl₂, 1 mM DTT, 0.5 U RNase Inhibitor, 1 mM ATP, 10 U T4 RNA Ligase) inkubierte für 2h bei 25° C. Dann wurde ein Pufferwechsel zu Wasser mittels eines Amicons (Merck) (100 kDa cut-off) durchgeführt.

Die erhaltenen Adapter-Ligationsprodukte (Adapter Oligonukleotid + mRNA) wurden im Anschluss über eine reverse Transkriptase - Reaktion (RT-PCR) amplifiziert. Für die RT-PCR fand das SuperScript^{™} Kit entsprechend der Herstellerangaben Verwendung (Thermo Fisher Scientific). Für die Reaktion wurden spezifische Primer verwendet, die direkt 5' vor dem Poly A (Forward, SEQ ID NO:20) und an der Adaptersequenz binden (Reverse, SEQ ID NO:21). Die aus der RT-PCR erhaltenen Produkte wurden über ein PCR Purification Kit von Thermo Fisher nach Herstellerangaben gereinigt und die Proben anschließend mittels Elektrophorese in einem 15 % Polyacrylamid Gel analysiert. Dazu wurde die aufgetrennte DNA mittels Midori Green (Biozym) gefärbt und in einer Geldokumentationsanlage (BioRad) visualisiert (siehe Abbildung 3).

Abbildung 3 zeigt die Analyse des Poly A-Anhangs mittels Acrylamid-Gelelektrophorese. In Spur 1 wurde der Marker, GeneRuler^{™} 1 kb DNA Ladder (Thermo Fisher Scientifc) aufgetragen. In den folgenden 3 Spuren wurden die RT-PCR-Produkte für die RNAs, welche mit unterschiedlich linearisierten DNA-Templaten hergestellt wurden, aufgetrennt (SpCas9-linearisiert (Spur2), mit Typ IIS Restriktionsenzym linearisiert (Spur3) und PCR-Produkt (Spur4)).

In Abbildung 3 ist zu erkennen, dass in allen 3 RNA-Proben die gleichen Produkte mit der exakt gleichen Größenverteilung der RT-PCR-Produkte erhalten wurden. Da die Ursprungs-RNAs bereits sehr große Ähnlichkeiten aufweisen (vgl. Abbildung 2) ist davon auszugehen, dass die RT-PCR hier die gleichen bzw. sehr ähnliche Template als Ausgangsmaterial hatte. Die Gelmuster zeigen also, dass mit der erfindungsgemäßen Methode nicht nur pDNA effektiv linearisiert werden kann, sondern dass die pDNA auch an der korrekten, vorher definierten Stelle linearisiert wird und der gewünschte unverdeckte freie Poly A-Anhang entsteht.

### Beispiel 3: Promotor-Design für eine induzierbare Expression von Cas9

Da eine konstitutive Expression einer Cas-Endonuklease, beispielsweise Cas9, in einer Bakterienzelle *in vivo* unspezifische DNA-Schnittereignisse an verschiedenen Stellen sowohl in Plasmiden als auch im Chromosom einer Bakterienzelle verursachen kann, und so die Bakterienzelle als Plasmidproduzent schädigen kann, ist eine induzierbare Expression der Cas-Endonuklease, beispielsweise Cas9, unter einem regulierbaren Promoter mit möglichst geringer Basalexpression bevorzugt.

Für die induzierbare Expression der Cas-Nuklease und damit der zu einem gewünschten Kultivierungszeitpunkt via Induktor-Zugabe "anschaltbaren" *in vivo*-Linearisierung des Plasmidprodukts ist zusätzlich eine Begrenzung der induzierbaren Expressionshöhe bevorzugt. Eine zu hohe Expression einer Cas-Nuklease in einer kurzen Zeitspanne kann ähnlich negative Effekte auf die Plasmid-DNA und die Produzentenzelle haben wie eine hohe Basalexpression über eine längere Zeitspanne. Daher sind Promotoren besonders bevorzugt, die sowohl eine geringe Basalexpression als auch nach einer Induktion eine moderate Expressionsstärke aufweisen.

Der pTac1 Promoter (de Boehr, 1983, Proceedings of the National Academy of Sciences 80(1):21-5), ein Hybrid aus trp- und lac-Promoter, besitzt vom ursprünglichen Lac-Operon nur noch eine verbliebene Operator-Site LacO1 an Position +1 (Transkriptionsstart) zum Binden des Repressorproteins LacI. Das Repressorprotein LacI selbst, bestehend aus einer N-terminalen "headpiece" DNA-Bindeeinheit, einer hinge-Region, einem Core-Bereich mit N-terminalen und C-terminalen Subdomänen inkl. Lactose-Bindungsbereich und Dimerisierungsmotiv, sowie einem C-terminalen Mini-Zipper für die Tetramerisierung, assoziiert zu einem Dimer von Dimeren. Jedes LacI-Dimer bindet eine 21 bp Duplex DNA-Operator Sequenz LacO (Lewis 2011. J Mol Biol. 409, 14-27). Vom assoziierten LacI-Tetramer werden im nativen Lac-Operon zwei Duplex-DNA-Operator-Sites (LacO1 und LacO3) simultan gebunden, was im DNA-Strang einen sogenannten *"repression loop"* für eine maximale Repression ausbildet (Oehler et al. 2006, Nucleic Acids Res. 34, 606-612).

In WAC003 (SEQ ID NO:6) und WAC003-LacIdel (SEQ ID NO:7) wurde eine synthetische LacO-Bindungsstelle über die -35 Region des pTac1 Promoters eingefügt, ohne die Nukleotidsequenzen der -35 und -10 Promoter-Regionen, den Abstand (Spacer) zwischen beiden Regionen oder die Nukleotidsequenz des Spacers selbst zu verändern. WAC003 interagiert mit dem nativen, also zur Tetramerisierung fähigem LacI-Repressorprotein. Im Konstrukt WAC003-LacIdel wurde das Gen *lac*I durch eine Variante LacIdel ersetzt, die in Folge eines Nukleotid-Frameshifts am C-Terminus des kodierten LacI Proteins eine andere Aminosäuresequenz ausbildet und so das LacI-Tetramer nicht mehr ausbilden kann, sondern nur noch das LacI-Dimer.

Die Basalexpression (Repression) und die nach Induktor-Zugabe erreichbare Expression (Derepression) wurde mit dem Reporterprotein GFP geprüft. GFP als zytoplasmatisch lokalisiertes Protein stellt selbst keine Belastung für die Zellen dar und eignet sich somit gut als Referenzprotein zur Messung der Expressionsleistung. Das Gen für GFP wurde unter die Kontrolle der WAC003 und WAC003-LacIdel-Promotorkonstrukte gestellt, auf ein Plasmid mit ColE1 *origin of replication* kloniert, die Plasmide in einen *E. coli* K12 Stamm transformiert und die Basalexpression sowie die induzierbare Expression in Schüttelkultur in LB-Medium wie folgt getestet.

Aus Vorkulturen wurden Hauptkulturen auf OD₆₀₀ = 0,1 angeimpft. Nach 2 h, bei einer korrelierenden Zelldichten von OD₆₀₀ ~0,4, erfolgte keine (= Basalexpression) bzw. die Zugabe von 0,1 mM IPTG (= Induktion). Nach weiteren 24 h Kultivierung wurde die Menge an exprimierten GFP vermessen (Anregung 380 nm, Emission 500 nm) und auf die jeweils zugrunde liegende Zelldichte OD₆₀₀ der Kultur normiert. Als Kontrolle wurde eine Zelllinie mit dem pTac1 Promoter (hohe Basalexpression, hohe induzierbare Expressionsstärke) mitgeführt.

Abbildung 4 zeigt die Basalexpression und Expressionsstärke der WAC003 und WAC003-LacIdel-Promotorkonstrukte im Vergleich zur Tac1 Ausgangsvariante. Die WAC003 Variante zeigte eine gegenüber Tac1 deutlich verringerte Basalexpression. Nach IPTG-Zugabe war die Derepression des WAC003 Promoters ebenfalls deutlich reduziert (nur ~16 % GFP/OD₆₀₀ verglichen mit Tac1). Im WAC003-LacIdel Konstrukt ist die Basalexpression ebenfalls niedrig, die induzierbare Expressionshöhe ist zwar etwas höher als bei WAC003, aber immer noch sehr niedrig im Vergleich zu Tac1. Die Promotoren WAC003 und WAC003-LacIdel eignen sich somit sehr gut für die induzierbare Expression einer Cas-Nuklease, beispielsweise Cas9, welche *in vivo* zur spezifischen Linearisierung von Plasmid-DNA exprimiert werden soll.

### Beispiel 4: In vivo-Induktion von Cas9 und Aufreinigung von linearer DNA nach Inkubation

Zur Analyse der *in* vivo-Funktionalität des in Beispiel 1 und Beispiel 2 beschriebenen Systems, wurden sowohl das Gen für SpCas9 kodiert (SEQ ID NO:2), als auch das Konstrukt für die Führungs-RNA (SEQ ID NO:1) im selben Plasmid (pWAC003, SEQ ID NO:5) jeweils unter den induzierbaren Promoter WAC003-LacIDel (SEQ ID NO:7) kloniert. Dabei wurden zwei SpCas9-spezifische Primer verwendet, (SEQ ID NO:22 und 23) um das *Sp_cas9-*Gen für die Klonierung aus dem Plasmid pCas9-ts1 (SEQ ID NO:24) zu amplifizieren. Das resultierende PCR-Produkt wurde über *Eco*RI und *XbaI* in das Plasmid pWAC003 (SEQ ID NO:5) hinter den Promotor WAC003-LacIDel (SEQ ID NO:7) kloniert. Die Cas9-Expressionskassette des resultierende Plasmids pCas9 (SEQ ID NO:25) wurde mit spezifischen Primern (SEQ ID NO:26 undSEQ ID NO:27) sequenziert (Sequiserve), um die gewünschte Sequenz zu bestätigen.

Da in einem *in* vivo-System die Herstellung der Führungs-RNA nicht durch eine Run-Off Transkription, wie in Beispiel 2 beschrieben, durchgeführt werden kann, bedarf es einer Anpassung des RNA-Designs. Für die Erzeugung einer funktionalen Führungs-RNA muss auf die natürliche Form der RNA-Prozessierung von CRISPR-Cas9 Systemen zurückgegriffen werden. Das heißt, es wird ein minimaler CRISPR-Array und eine tracrRNA bereitgestellt.

In das Plasmid pCas9 (SEQ ID NO:25) wurden eine tracrRNA-Sequenz (SEQ ID NO:28) sowie eine CRISPR-Sequenz (SEQ ID NO:29) eingefügt. Die Sequenzen SEQ ID NO:28 und SEQ ID NO:29 umfassen neben der tracrRNA-Sequenz bzw. der CRISPR-Sequenz jeweils eine WAC003-LacIdel-Promotorsequenz und eine Rho-unabhängige Terminator Sequenz, in welche die tracrRNA bzw. CRISPR-Sequenz eingebettet sind (5'-3'). Dafür wurden zunächst die beiden DNA-Fragmente jeweils über eine PCR hergestellt, für SEQ ID NO:28 wurden die Primer mit den SEQ ID NO:30 - 35 verwendet und für die SEQ ID NO:29 wurden die Primer mit den SEQ ID NO:36 - 41 genutzt. Die tracrRNA-Sequenz wurde über die beiden Restriktionsenzyme KpnI und *XbaI* in pCas9 kloniert, so dass sie 3' nach dem Cas9 Konstrukt liegt. In das entstandene Plasmid pCas9_tracrRNA (SEQ ID NO:42) wurde die CRISPR-Sequenz über die Schnittstellen *Xma*I und *XbaI* in 3' Position hinter die tracrRNA Sequenz kloniert. Die korrekte Assemblierung des entstandenen Plasmids pCas9_tracrRNA_CRISPR (SEQ ID NO:43) wurde mittels Sequenzierung überprüft, dazu wurde der spezifische Primer (SEQ ID NO:27) verwendet.

Abbildung 5 zeigt eine Schematische Darstellung des Plasmids pCas9_tracrRNA_CRISPR. Im Plasmid pCas9_tracrRNA_CRISPR sind alle drei Elemente Cas9, tracrRNA und CRISPR jeweils unter Kontrolle eines WAC003-LacIdel Promoters und verfügen zusätzlich über eine Rho-unabhängige Terminator Sequenz.

Das Plasmid pCas9_tracrRNA_CRISPR (SEQ ID NO:43) wurde zusammen mit pGFP1 (SEQ ID NO:11) in *E. coli* NEB10Beta transformiert. Nach Selektion auf LB-Ampicillin/Tetracyclin wurden Klone in 20 mL LB mit Antibiotika (100 mg/L Ampicillin oder 20 mg/L Tetracyclin) angeimpft und über Nacht bei 37 °C inkubiert (200 rpm). Mit den Übernachtkulturen wurde 100 mL frisches LB-Medium mit Ampicillin bzw. Tetracyclin (100 mg/L bzw. 20 mg/L) angeimpft. Die resultierende Hauptkultur wurde bis zu einer OD₆₀₀ₙₘ von 0.8 unter Schütteln bei 37°C inkubiert. Bei Erreichen der Ziel-OD wurde IPTG auf eine finale Konzentration von 0,5 mM hinzugefügt, um die Expression der Cas9, der CRISPR-Kasette und der tracrRNA gleichzeitig zu induzieren. Aufgrund der Co-Expression dieser Elemente wird das Plasmid pGFP1 spezifisch gespalten. 2 h nach Induktion wurden die Zellen mittels Zentrifugation sedimentiert. Die DNA im Überstand (linearisiertes pGFP1 und zirkuläres pCas9_tracrRNA_crRNA) wurde anschließend nach Herstellerangaben mit dem Gene Jet Plasmid Kit (Thermo Fisher Scientific) isoliert und gereinigt.

Die Zusammensetzung der isolierten DNA und der Anteil des linearisierten pGFP1 wurde mittels Agarose Gelelektrophorese und Kapillarelektrophorese analysiert. Für die Agarose Gelelektrophorese wurde die isolierte DNA über ein 1,5%-iges Agarose-Gel getrennt und anschließend mittels Midori Green (Nippon Genetics Europe) und einer Geldokumentationsanalage (BioRad) visualisiert.

Die Verwendbarkeit der linearisierten DNA als Templat für die Herstellung von mRNA wurde wie in Beispiel 2 beschrieben über eine *in* vitro-Transkription überprüft. Die erhaltenen RNAs wurden mittels Polyacrylamid Gelelektrophorese analysiert und die Poly A-Anhänge der mRNAs wurden über die beschriebene Methode mit T4 RNA Ligase 1, RT-PCR analysiert.

### Beispiel 5: Inkorporation des cas9-CRISPR-tracrRNA Konstruktes auf dem Chromosom eines ausgewählten E. coli Stamms

Das in Beispiel 4 beschriebene CRISPR-Cas Konstrukt pCas9_tracrRNA_CRISPR (SEQ ID NO:43) wurde in das Chromosom von *E. coli* NEB10Beta integriert. Für die Integration wurde ein intergenischer Bereich des Genlokus von *atpI* gewählt. Für die Integration des Cas9-gRNA Konstruktes wurde die Gen-Deletions-Methode mittels λ-Red Rekombination nach Datsenko und Wanner (Datsenko et al. 2000, Proc. Natl. Acad. Sci. U S A. 97: 6640 - 5) genutzt.

Für die Integration des Cas9-CRISPR-tracrRNA Konstrukts in den intergenischen Bereich des atpI Lokus in das Genom des verwendeten *E. coli* NEB10Beta Stammes wurde zunächst das zu integrierende Konstrukt entworfen. Dieses enthält neben dem in Beispiel 4 beschriebenen cas9_tracrRNA_CRISPR Konstrukt (SEQ ID NO:43) eine Chloramphenicol Resistenzkassette (cat), zwei FRT *sites,* welche die cat Kassette flankieren, sowie jeweils einen flankierenden homologen Bereich zum Integrationsort. Das Konstrukt (SEQ ID NO:44) für die genomische Integration mittels λ-Red Rekombination ist schematisch in Abbildung 6 dargestellt. Zwei jeweils 150 bp lange homologe Bereiche zum Integrationsort flankieren das Konstrukt am 3' und am 5' Ende. Dazwischen befinden sich in 5' - 3' Orientierung das *cas9*_tracrRNA_CRISPR Konstrukt, eine FRT *site,* die cat Kassette und eine weitere FRT *site.*

Die cat Kassette dient zur späteren Selektion, um eine erfolgreiche Integration zu identifizieren. Die FRT sites werden im späteren Verlauf genutzt, um mit Hilfe einer Rekombinase die *cat* Kassette wieder aus dem Genom zu entfernen. Die homologen Regionen, welche das gesamte Konstrukt flankieren, sind 150 Basenpaare lang und dienen zur Integration am Zielort. Das Einbauen des Konstruktes in das Genom findet durch homologe Rekombination statt.

Das vollständige Konstrukt wurde von GeneArt synthetisiert und in den Vektor pMX Vektor (GeneArt) kloniert. Aus diesem Plasmid wurde das Konstrukt über Restriktionsenzyme (XhoI) herausgeschnitten und mittels GeneJet Gel-Extraktionskit (Thermo Fisher Scientific) nach Herstellerangaben gereinigt.

Um die homologe Rekombination für die Integration zu vermitteln, wurde das Lambda Red System verwendet, welches auf dem Plasmid pKD46 (CGSC: 7736) kodiert ist. Das Plasmid wurde in NEB10Beta Zellen transformiert und anschließend werden nach den Angaben von Datsenko und Wanner ((Datsenko et al. 2000, Proc. Natl. Acad. Sci. U S A. 97: 6640 - 5) kompetente Zellen hergestellt. Diese kompetenten Zellen wurden mit dem gereinigten Integrationskonstrukt transformiert. Die Selektion auf Integration der Chloramphenicol-Resistenzkassette *(cat* = Chloramphenicol-Acetyltransferase) und damit auch des Cas9 bzw. Führungs-RNA-Konstrukts in das Chromosom erfolgte auf LB-AgarPlatten, die 20 mg/l Chloramphenicol enthalten. Die Kontrolle bzw. Bestätigung der Integration an der gewünschten Position im Chromosom erfolgte mittels PCR unter Verwendung der Oligonukleotide Check Fwd (SEQ ID NO:54) und Check Rev (SEQ ID NO:55) und chromosomaler DNA der Chloramphenicol-resistenten Zellen als Matrize. Als Negativkontrolle wurde die PCR auch mit chromosomaler DNA des Ausgangsstamms *E. coli* NEB10Beta durchgeführt. Auf diese Weise wurden *E*. coli-Zellen erhalten, bei denen das Cas9- bzw. Führungs-RNA-Konstrukt ins Genom integriert ist.

Anschließend wurden die Zellen nach der von Datsenko und Wanner (s.o.) beschriebenen Prozedur von dem Plasmid pKD46 kuriert und der auf diese Weise erzeugte Stamm wurde als *E. coli* NEB10Beta *atpI*::*cas9*-CRISPR-tracrRNA-*cat* bezeichnet. Die Entfernung der Chloramphenicol-Resistenzkassette aus dem Chromosom erfolgte nach der Vorschrift von Datsenko und Wanner (s.o.) mit Hilfe des Plasmids pCP20 (CGSC: 7629), welches für das FLP-Rekombinase-Gen kodiert. Der schließlich mit dieser Methode erhaltene Chloramphenicol sensitive Cas9-CRISPR-tracrRNA enthaltende Stamm wurde als *E. coli* NEB10Beta *atpI*::*cas9*-CRISPR-tracrRNA bezeichnet. Die erfolgreiche Integration des Konstruktes (SEQ ID NO:44) wurde abschließend über Sequenzierung, unter Verwendung verschiedener Primer (SEQ ID NO:45-54), überprüft.

In den neuen Stamm *(E. coli* NEB10Beta atpI: : cas9-CRISPR-tracrRNA) wurde anschließend zur Prüfung des Systems das Plasmid pGFP1 (SEQ ID NO:11) transformiert. Nach der Regeneration und Selektion (über Nacht auf LB-Ampicillin Agar Platten) wurde eine Kolonie ausgewählt, mit welcher zunächst eine Übernachtkultur in 20 mL LB-Amp angesetzt wurde (37 °C, 200 rpm). Diese Vorkultur diente zum Animpfen der 100 mL Hauptkultur. Die resultierende Hauptkultur wurde bis zu einer OD₆₀₀ₙₘ von 0.8 inkubiert. Bei Erreichen der Ziel-OD wurde IPTG hinzugefügt, um die Expression sowohl von Cas9 als auch der Führungs-RNA zu starten. Nach 2h unter induzierenden Bedingungen wurde die Kultur mittels Zentrifugation geerntet. Die Plasmid-DNA in den Zellen wurde anschließend nach Herstellerangaben mit dem Gene Jet Plasmid Kit (Thermo Fisher Scientific) isoliert und gereinigt.

Die isolierte Plasmid-DNA wurde mittels Agarose-Gelelektrophorese und Kapillarelektrophorese analysiert. Für die Gelelektrophorese wurde die isolierte Plasmid-DNA mittels eines 1,5%-igen Agarose-Gels entsprechend der Größe und Eigenschaft (linarisiert, coiled) getrennt und anschließend mittels Midori Green (Nippon Genetics Europe) an einer Geldokumentationsanalage (BioRad) visualisiert. Die Verwendbarkeit der DNA für die Herstellung von mRNA wurde wie in Beispiel 2 beschrieben über eine *in* vitro-Transkription geprüft.

## Patentansprüche

1. Verfahren zur Herstellung eines linearen doppelsträngigen DNA-Moleküls,
worin das lineare DNA-Molekül glatte Enden aufweist, und worin das lineare DNA-Molekül eine Poly T-Sequenz am 5' Ende des Matrizen-Strangs aufweist, umfassend die Schritte:
(1) Bereitstellen eines doppelsträngigen zirkulären DNA-Moleküls, umfassend auf dem Matrizen-Strang in der 3'- zu 5'-Richtung:
(i) eine Templat-Sequenz, die operativ mit einem RNA-Polymerase-Promotor verbunden ist,
(ii) eine Poly T-Sequenz,
(iii)eine Nukleotidsequenz, dargestellt durch N₁N₂N₃, worin N₁ bis N₃ jeweils unabhängig voneinander G, T, A oder C darstellen, und
(iv) ein Protospacer Adjacent Motif (PAM),
(2) Bereitstellen einer Führungs-RNA, umfassend eine Region, die komplementär ist zum 5'-Ende der Poly T-Sequenz (ii) und zur Nukleotidsequenz (iii), und eine Region, die mit einer Cas-Nuklease interagiert,
(3) Bereitstellen einer Cas-Nuklease vom Typ II,
(4) In Kontakt bringen des doppelsträngigen zirkulären DNA-Moleküls mit der Führungs-RNA und der CAS-Nuklease.

2. Verfahren nach Anspruch 1, worin die Poly T-Sequenz (ii) aus zwischen etwa 40 bis etwa 250 T-Nukleotiden besteht, bevorzugt aus zwischen etwa 100 und etwa 140 T-Nukleotiden, und besonders bevorzugt aus etwa 120 T-Nukleotiden.

3. Verfahren nach Anspruch 1 oder 2, worin die Region der Führungs-RNA, die komplementär ist zum 5'-Ende der Poly T-Sequenz (ii)und zur Nukleotidsequenz (iii) die Sequenz 5'-G(A)ₙN₄N₅N₆-3' aufweist, worin n eine Zahl zwischen 17 und 23 ist, worin N₄ bis N₆ jeweils unabhängig G, U, A oder C darstellt, und worin die Nukleotide N₄N₅N₆ komplementär sind zu den Nukleotiden N₁N₂N₃ aus (iii).

4. Verfahren nach einem der Ansprüche 1 bis 3, worin es sich bei der Cas-Nuklease vom Typ II um Cas9 handelt, bevorzugt ausgewählt aus Cas9 aus *Streptococcus pyogenes* mit der SEQ ID NO:2 oder einer dazu zu mindestens 70% homologen Sequenz, Cas9 aus *Streptococcus aureus* mit der SEQ ID NO:3 oder einer dazu zu mindestens 70% homologen Sequenz und Cas9 aus *Streptococcus thermophilus* mit der SEQ ID NO:4 oder einer dazu zu mindestens 70% homologen Sequenz.

5. Verfahren nach einem der Ansprüche 1 bis 4, worin es sich bei der Cas-Nuklease vom Typ II um Cas9 aus *Streptococcus pyogenes* mit der SEQ ID NO:2 oder einer dazu zu mindestens 70% homologen Sequenz handelt.

6. Verfahren nach einem der Ansprüche 1 bis 5, worin das PAM die Sequenz 5'-NGG-3' aufweist, wobei N jeweils unabhängig ausgewählt ist aus G, C, A und T.

7. Verfahren nach einem der Ansprüche 1 bis 6,
worin die Poly T-Sequenz (ii) aus etwa 120 T-Nukleotiden besteht,
worin die Region der Führungs-RNA, die komplementär ist zum 5'-Ende der Poly T-Sequenz (ii)und zur Nukleotidsequenz (iii) die Sequenz 5'-G(A)ₙ - N₄N₅N₆-3' aufweist, worin n gleich 17 ist, worin N₄ bis N₆ jeweils unabhängig G, U oder C darstellt,
worin das PAM die Sequenz 5'-NGG-3' aufweist, wobei N jeweils unabhängig ausgewählt ist aus G, C, A und T, und worin es sich bei der Cas-Nuklease vom Typ II um Cas9 aus *Streptococcus pyogenes* mit der SEQ ID NO:2 oder einer dazu zu mindestens 70% homologen Sequenz handelt.

8. Verfahren nach einem der Ansprüche 1 bis 7, **dadurch gekennzeichnet, dass** das Bereitstellen der Führungs-RNA durch die Expression einer Nukleotidsequenz, kodierend für die Führungs-RNA erfolgt.

9. Verfahren nach einem der Ansprüche 1 bis 8, **dadurch gekennzeichnet, dass** das Bereitstellen der Cas-Nuklease vom Typ II durch die Expression einer Nukleotidsequenz kodierend für die Cas-Nuklease erfolgt.

10. Verfahren nach einem der Ansprüche 8 oder 9, **dadurch gekennzeichnet, dass** die Nukleotidsequenz kodierend für die Führungs-RNA und/oder die Nukleotidsequenz kodierend für die Cas-Nuklease operativ mit einem induzierbaren Promotor verbunden ist.

11. Verfahren nach Anspruch 10, worin der induzierbare Promotor ein durch Lactose-induzierbarer Promotor ist, insbesondere der Promotor WAC003 mit der Sequenz SEQ ID NO:6 oder der Promotor WAC003-LacDel mit der Sequenz SEQ ID NO:7.

12. Verfahren nach einem der Ansprüche 8-11, **dadurch gekennzeichnet, dass** die Nukleotidsequenz kodierend für die Führungs-RNA und/oder die Nukleotidsequenz kodierend für die Cas-Nuklease in das Genom von *E. coli* integriert ist.

13. Verwendung einer Cas-Nuklease vom Typ II zur Linearisierung eines doppelsträngigen, zirkulären DNA-Moleküls, worin das lineare DNA-Molekül auf dem Matrizen-Strang in der 3'- zu 5'-Richtung umfasst:
(i) eine Templat-Sequenz, die operativ mit einem RNA-Polymerase-Promotor verbunden ist,
(ii) eine Poly T-Sequenz,
(iii)eine Nukleotidsequenz, dargestellt durch N₁N₂N₃, worin N₁ bis N₃ jeweils unabhängig voneinander G, T, A oder C darstellen, und
(iv) ein Protospacer Adjacent Motif (PAM).

14. Verwendung eines rekombinanten Mikroorganismus, worin die Nukleotidsequenz kodierend für eine Führungs-RNA und/oder die Nukleotidsequenz kodierend für eine Cas-Nuklease vom Typ II in das Genom integriert ist, zur Herstellung eines linearen doppelsträngigen DNA-Moleküls, worin das lineare DNA-Molekül glatte Enden aufweist, und worin das lineare DNA-Molekül eine Poly T-Sequenz am 5' Ende des Templat-Stranges aufweist.

15. Verwendung nach Anspruch 14, worin der rekombinante Mikroorganismus *E. coli* ist, bevorzugt ein *E. coli* K12 Stamm, besonders bevorzugt *E. coli* NEB10Beta.

## Claims

1. Method for producing a linear double-stranded DNA molecule,
the linear DNA molecule having blunt ends, and
the linear DNA molecule having a poly T sequence at the 5' end of the template strand, comprising the steps of:
(1) providing a double-stranded circular DNA molecule comprising on the template strand in the 3' to 5' direction:
(i) a template sequence operatively linked to an RNA polymerase promoter,
(ii) a poly T sequence,
(iii) a nucleotide sequence described by N₁N₂N₃, where N₁ to N₃ are each independently G, T, A or C, and
(iv) a protospacer adjacent motif (PAM),
(2) providing a guide RNA comprising a region which is complementary to the 5' end of the poly T sequence (ii) and to the nucleotide sequence (iii) and a region which interacts with a Cas nuclease,
(3) providing a type II Cas nuclease,
(4) contacting the double-stranded circular DNA molecule with the guide RNA and the CAS nuclease.

2. Method according to Claim 1, wherein the poly T sequence (ii) consists of between about 40 and about 250 T nucleotides, preferably between about 100 and about 140 T nucleotides, and particularly preferably about 120 T nucleotides.

3. Method according to Claim 1 or 2, wherein the region of the guide RNA which is complementary to the 5' end of the poly T sequence (ii) and to the nucleotide sequence (iii) has the sequence 5'-G(A)ₙN₄N₅N₆-3', where n is a number between 17 and 23, where N₄ to N₆ are each independently G, U, A or C, and where the nucleotides N₄N₅N₆ are complementary to the nucleotides N₁N₂N₃ from (iii) .

4. Method according to any of Claims 1 to 3, wherein the type II Cas nuclease is Cas9, preferably selected from Cas9 from Streptococcus pyogenes having SEQ ID NO:2 or a sequence at least 70% homologous thereto, Cas9 from Streptococcus aureus having SEQ ID NO:3 or a sequence at least 70% homologous thereto and Cas9 from Streptococcus thermophilus having SEQ ID NO:4 or a sequence at least 70% homologous thereto.

5. Method according to any of Claims 1 to 4, wherein the type II Cas nuclease is Cas9 from Streptococcus pyogenes having SEQ ID NO:2 or a sequence at least 70% homologous thereto.

6. Method according to any of Claims 1 to 5, wherein the PAM has the sequence 5'-NGG-3', where N is independently selected from G, C, A and T.

7. Method according to any of Claims 1 to 6,
wherein the poly T sequence (ii) consists of about 120 T nucleotides,
wherein the region of the guide RNA which is complementary to the 5' end of the poly T sequence (ii) and to the nucleotide sequence (iii) has the sequence 5'-G(A)ₙ - N₄N₅N₆-3', where n is equal to 17, where N₄ to N₆ are each independently G, U or C,
wherein the PAM has the sequence 5'-NGG-3', where N is independently selected from G, C, A and T, and
wherein the type II Cas nuclease is Cas9 from Streptococcus pyogenes having SEQ ID NO:2 or a sequence at least 70% homologous thereto.

8. Method according to any of Claims 1 to 7, **characterized in that** the providing of the guide RNA is achieved by the expression of a nucleotide sequence encoding the guide RNA.

9. Method according to any of Claims 1 to 8, **characterized in that** the providing of the type II Cas nuclease is achieved by the expression of a nucleotide sequence encoding the Cas nuclease.

10. Method according to either of Claims 8 and 9, **characterized in that** the nucleotide sequence encoding the guide RNA and/or the nucleotide sequence encoding the Cas nuclease is/are operatively linked to an inducible promoter.

11. Method according to Claim 10, wherein the inducible promoter is a lactose-inducible promoter, in particular the promoter WAC003 having the sequence SEQ ID NO:6 or the promoter WAC003-LacDel having the sequence SEQ ID NO:7.

12. Method according to any of Claims 8-11, **characterized in that** the nucleotide sequence encoding the guide RNA and/or the nucleotide sequence encoding the Cas nuclease has/have been integrated into the genome of E. coli.

13. Use of a type II Cas nuclease for linearization of a double-stranded, circular DNA molecule, the linear DNA molecule comprising on the template strand in the 3' to 5' direction:
(i) a template sequence operatively linked to an RNA polymerase promoter,
(ii) a poly T sequence,
(iii) a nucleotide sequence described by N₁N₂N₃, where N₁ to N₃ are each independently G, T, A or C, and
(iv) a protospacer adjacent motif (PAM).

14. Use of a recombinant microorganism in which the nucleotide sequence encoding a guide RNA and/or the nucleotide sequence encoding a type II Cas nuclease has/have been integrated into the genome for production of a linear double-stranded DNA molecule, the linear DNA molecule having blunt ends, and the linear DNA molecule having a poly T sequence at the 5' end of the template strand.

15. Use according to Claim 14, wherein the recombinant microorganism is E. coli, preferably an E. coli K12 strain, particularly preferably E. coli NEB10Beta.

## Revendications

1. Procédé de préparation d'une molécule d'ADN linéaire double brin,
dans lequel la molécule d'ADN linéaire présente des extrémités lisses et
dans lequel la molécule d'ADN linéaire présente une séquence poly T en l'extrémité 5' du brin de matrice, comprenant les étapes de :
(1) mise à disposition d'une molécule d'ADN double brin circulaire, comprenant sur le brin de matrice, dans le sens 3' à 5':
(i) une séquence de matrice qui est reliée fonctionnellement à un promoteur d'ARN polymérase,
(ii) une séquence poly T,
(iii) une séquence nucléotidique, représentée par N₁N₂N₃, dans laquelle N₁ à N₃ représentent, à chaque fois indépendamment les uns des autres, G, T, A ou C et
(iv) un motif de reconnaissance du proto-espaceur (Protospacer Adjacent Motif - PAM),
(2) mise à disposition d'un ARN guide comprenant une région qui est complémentaire à l'extrémité 5' de la séquence poly T (ii) et à la séquence nucléotidique (iii) et une région qui interagit avec une nucléase Cas,
(3) mise à disposition d'une nucléase Cas de type II,
(4) mise en contact de la molécule d'ADN double brin circulaire avec l'ARN guide et la nucléase CAS.

2. Procédé selon la revendication 1, dans lequel la séquence poly T (ii) est constituée par entre environ 40 et environ 250 nucléotides T, de préférence par entre environ 100 et environ 140 nucléotides T et de manière particulièrement préférée par environ 120 nucléotides T.

3. Procédé selon la revendication 1 ou 2, dans lequel la région de l'ARN guide, qui est complémentaire à l'extrémité 5' de la séquence poly T (ii) et à la séquence nucléotidique (iii), présente la séquence 5'-G(A)ₙN₄N₅N₆-3', dans laquelle n représente un nombre entre 17 et 23, dans laquelle N₄ à N₆ représentent, à chaque fois indépendamment, G, U, A ou C et dans laquelle les nucléotides N₄N₅N₆ sont complémentaires aux nucléotides N₁N₂N₃ de (iii).

4. Procédé selon l'une des revendications 1 à 3, dans lequel il s'agit, pour la nucléase Cas du type II, de Cas9, de préférence choisie parmi Cas9 de Streptococcus pyogenes présentant la séquence SEQ ID NO:2 ou une séquence homologue à raison d'au moins 70% par rapport à celle-ci, de Cas9 de Streptococcus aureus présentant la séquence SEQ ID NO:3 ou une séquence homologue à raison d'au moins 70% par rapport à celle-ci et de Cas9 de Streptococcus thermophilus présentant la séquence SEQ ID NO:4 ou une séquence homologue à raison d'au moins 70% par rapport à celle-ci.

5. Procédé selon l'une des revendications 1 à 4, dans lequel il s'agit, pour la nucléase Cas de type II, de Cas9 de Streptococcus pyogenes présentant la séquence SEQ ID NO:2 ou une séquence homologue à raison d'au moins 70% par rapport à celle-ci.

6. Procédé selon l'une des revendications 1 à 5, dans lequel le PAM présente la séquence 5'-NGG-3', N étant choisi à chaque fois indépendamment parmi G, C, A et T.

7. Procédé selon l'une des revendications 1 à 6,
dans lequel la séquence poly T (ii) est constituée par environ 120 nucléotides T,
dans lequel la région de l'ARN guide, qui est complémentaire à l'extrémité 5' de la séquence poly T (ii) et à la séquence nucléotidique (iii), présente la séquence 5'-G(A)ₙ - N₄N₅N₆-3', dans laquelle n vaut 17, dans laquelle N₄ à N₆ représentent, à chaque fois indépendamment, G, U ou C,
dans lequel le PAM présente la séquence 5'-NGG-3', N étant choisi à chaque fois indépendamment parmi G, C, A et T et
dans lequel il s'agit, pour la nucléase Cas de type II, de Cas9 de Streptococcus pyogenes présentant la séquence SEQ ID NO:2 ou une séquence homologue à raison d'au moins 70% par rapport à celle-ci.

8. Procédé selon l'une des revendications 1 à 7, **caractérisé en ce que** la mise à disposition de l'ARN guide est effectuée par l'expression d'une séquence nucléotidique, codant pour l'ARN guide.

9. Procédé selon l'une des revendications 1 à 8, **caractérisé en ce que** la mise à disposition de la nucléase Cas de type II est effectuée par l'expression d'une séquence nucléotidique, codant pour la nucléase Cas.

10. Procédé selon l'une des revendications 8 ou 9, **caractérisé en ce que** la séquence nucléotidique codant pour l'ARN guide et/ou la séquence nucléotidique codant pour la nucléase Cas est/sont reliée(s) fonctionnellement à un promoteur pouvant être induit.

11. Procédé selon la revendication 10, dans lequel le promoteur pouvant être induit est un promoteur pouvant être induit par le lactose, en particulier le promoteur WAC003 présentant la séquence SEQ ID NO:6 ou le promoteur WAC003-LacDel présentant la séquence SEQ ID NO:7.

12. Procédé selon l'une des revendications 8 à 11, **caractérisé en ce que** la séquence nucléotidique codant pour l'ARN guide et/ou la séquence nucléotidique codant pour la nucléase Cas est/sont intégrée(s) dans le génome d'E. coli.

13. Utilisation d'une nucléase Cas du type II pour la linéarisation d'une molécule d'ADN double brin, circulaire, dans laquelle la molécule d'ADN linéaire sur le brin de matrice dans le sens 3'- à 5' comprend :
(i) une séquence de matrice qui est reliée fonctionnellement à un promoteur d'ARN polymérase,
(ii) une séquence poly T,
(iii) une séquence nucléotidique, représentée par N₁N₂N₃, dans laquelle N₁ à N₃ représentent, à chaque fois indépendamment les uns des autres, G, T, A ou C et
(iv) un motif de reconnaissance du proto-espaceur (Protospacer Adjacent Motif - PAM).

14. Utilisation d'un microorganisme recombinant, dans lequel la séquence nucléotidique codant pour un ARN guide et/ou la séquence nucléotidique codant pour une nucléase Cas du type II est/sont intégrée(s) dans le génome, pour la préparation d'une molécule d'ADN double brin linéaire, la molécule d'ADN linéaire présentant des extrémités lisses et la molécule d'ADN linéaire présentant une séquence poly T en l'extrémité 5' du brin de matrice.

15. Utilisation selon la revendication 14, dans laquelle le microorganisme recombinant est E. coli, de préférence une souche d'E. coli K12, de manière particulièrement préférée E. coli NEB10Beta.
